# EUROPEAN PATENT SPECIFICATION

(11) **EP 3 634 932 B1**
(45) Date of publication and mention of the grant of the patent: **24.07.2024**
(21) Application number: 18735061.6
(22) Date of filing: 07.06.2018
(51) Int. Cl.: C07C 51/00, C07C 57/04, C08G 8/08, C07C 51/15, C08G 8/10

(54) **HIGH POROSITY AROMATIC RESINS AS PROMOTERS IN ACRYLATE PRODUCTION FROM COUPLING REACTIONS OF OLEFINS AND CARBON DIOXIDE**
HOCHPORÖSE AROMATISCHE HARZE ALS PROMOTOR IN DER ACRYLATPRODUKTION AUS KOPPLUNGSREAKTIONEN VON OLEFINEN UND KOHLENSTOFFDIOXID
RÉSINES AROMATIQUES À POROSITÉ ÉLEVÉE EN TANT QUE PROMOTEURS DANS LA PRODUCTION D'ACRYLATE À PARTIR DE RÉACTIONS DE COUPLAGE D'OLÉFINES ET DE DIOXYDE DE CARBONE

(30) Priority: 14.06.2017 US 201762519549 P
(43) Date of publication of application: 15.04.2020
(73) Proprietor: Chevron Phillips Chemical Company LP, The Woodlands, Texas 77380 (US)
(72) Inventor: IACONO, Pasquale, Tulsa, Oklahoma 74114 (US); HLAVINKA, Mark L., Tulsa, Oklahoma 74114 (US)
(74) Representative: Privett, Marianne Alice Louise
(86) International application number: PCT/US2018/036430
(87) International publication number: WO 2018/231623

(56) References cited:
- WO-A1-2015/173296
- US-A- 3 390 128

## Description

### TECHNICAL FIELD

This disclosure relates to routes of synthesis of acrylic acid and other α,β-unsaturated carboxylic acids, including catalytic methods.

### BACKGROUND

The majority of industrially synthesized chemical compounds are prepared from a limited set of precursors, whose ultimate sources are primarily fossil fuels. As these reserves diminish, it would be beneficial to use a renewable resource, such as carbon dioxide, which is a non-toxic, abundant, and economical Ci synthetic unit. The coupling of carbon dioxide with other unsaturated molecules holds tremendous promise for the direct preparation of molecules currently prepared by traditional methods not involving CO₂.

One could envision the direct preparation of acrylates and carboxylic acids through this method, when carbon dioxide is coupled with olefins. Currently, acrylic acid is produced by a two-stage oxidation of propylene. The production of acrylic acid directly from carbon dioxide and ethylene would represent a significant improvement due to the greater availability of ethylene and carbon dioxide versus propylene, the use of a renewable material (CO₂) in the synthesis, and the replacement of the two-step oxygenation process currently being practiced.

Therefore, what is needed are improved methods for preparing acrylic acid and other α,β-unsaturated carboxylic acids, including catalytic methods.

US 3,390,128 discloses synthetic resins and a method of making such resins by reacting formaldehyde with a bisphenol and a spaced polyphenol containing at least two phenolic groups per molecule, with the phenolic groups being joined by aliphatic hydrocarbons from about 12 to 30 carbon atoms. WO 2015/173296 discloses a process for preparing sodium acrylate by direct carboxylation of ethene with carbon dioxide (CO₂).

### SUMMARY OF THE DISCLOSURE

This summary is provided to introduce various concepts in a simplified form that are further described below in the detailed description. This summary is not intended to identify required or essential features of the claimed subject matter nor is the summary intended to limit the scope of the claimed subject matter.

This disclosure provides processes, including catalytic processes, for producing α,β-unsaturated carboxylic acids or salts thereof utilizing a porous crosslinked polyphenoxide resin comprising associated metal cations. Because the porous crosslinked polyphenoxide resin is insoluble and/or the reaction system is otherwise heterogeneous, these processes represent an improvement over homogeneous processes that result in poor yields and involve challenging separation and/or isolation procedures.

Conventional methods generally make isolation of the desired α,β-unsaturated carboxylic acid (e.g., acrylic acid) difficult. In contrast, the processes disclosed herein utilize a porous crosslinked polyphenoxide resin comprising associated metal cations, also referred to as simply a crosslinked polyphenolate or a crosslinked polyaryloxide resin, that generally provides a heterogeneous reaction mixture. When combined with a catalyst such as a nickel catalyst, ethylene and carbon dioxide can be coupled to form a metalalactone, and the porous crosslinked polyphenoxide resin can subsequently destabilize the metalalactone which eliminates a metal acrylate. By developing the disclosed heterogeneous system, there is now provided a distinct advantage in ease of separation of the desired product from the catalytic system. Moreover, the porous crosslinked polyphenoxide resins may result in surprisingly high yields of the desired α,β-unsaturated carboxylic acid, such as acrylic acid.

The porous crosslinked polyphenoxide resin may also be referred to as a co-catalyst, and typically has associated sodium or potassium ions. For example, the use of the heterogeneous, porous crosslinked sodium-appended cocatalyst is advantageous at least because [1] it provides a more facile means of separation the acrylate product from the catalyst system, because it is not soluble in the process diluent, [2] the polyphenoxide can be regenerated in a reactor setting by sodium base (e.g. NaOR or NaOH) treatment by hydroxyl deprotonation and/or by base (e.g. NaOR) absorption into the solid porous matrix, [3] it retains its robustness and structural integrity such that it does not degrade under the reaction conditions or regeneration conditions using sodium treatment e.g. NaOR or NaOH), and [4] its porous crosslinked structure allows for high sodium deposition density and facile sodium site access by incoming catalyst intermediates (such as metalalactones), as well as ease of regeneration.

According to an aspect, the crosslinked resin can be prepared by a templated polymerization process, which can provide its highly porous architecture with higher densities of sodium sites. The present invention provides a process for forming a porous crosslinked polyphenoxide resin, the process comprising:
a) in the presence of a basic particulate template, contacting at least one phenol compound, formaldehyde, and an aqueous base under polymerization conditions sufficient to form a templated crosslinked polyphenol resin comprising a crosslinked polyphenol resin in contact with the basic particulate template;
b) contacting the templated crosslinked polyphenol resin with an aqueous acid under pore forming conditions sufficient to remove the basic particulate template and form a porous crosslinked polyphenol resin; and
c) contacting the porous crosslinked polyphenol resin with a metal-containing base to form a promoter comprising a porous crosslinked polyphenoxide resin comprising associated metal cations.
Thus, by assembling the crosslinked polyphenol on a template, then removing the template, the resulting polyphenol and polyphenoxide can include a highly porous structure.

The present invention also provides a process for forming an α,β-unsaturated carboxylic acid or a salt thereof, the process comprising:
a) contacting
   1) a metalalactone compound;
   2) a diluent; and
   3) a promoter comprising a porous crosslinked polyphenoxide resin comprising associated metal cations to provide a reaction mixture; and
b) applying reaction conditions or process conditions to the reaction mixture suitable to induce a metalalactone elimination reaction to form the α,β-unsaturated carboxylic acid or the salt thereof.
According to this and other aspects of the disclosure, the metalalactone compound may also be described as a metalalactone comprising at least one ligand or simply a metalalactone, and these terms are used interchangeably to reflect that the metalalactone compound comprises at least one ligand in addition to the metalalactone moiety. Similarly, reference to a metalalactone ligand refers to any ligand of the metalalactone compound other than the metalalactone moiety.

The present invention also provides a process for forming an α,β-unsaturated carboxylic acid or a salt thereof, the process comprising:
a) contacting in any order
   1) a transition metal precursor compound comprising at least one first ligand;
   2) optionally, at least one second ligand;
   3) an olefin;
   4) carbon dioxide (CO₂);
   5) a diluent; and
   6) a promoter comprising a porous crosslinked polyphenoxide resin comprising associated metal cations to provide a reaction mixture; and
b) applying reaction conditions or process conditions to the reaction mixture suitable to form the α,β-unsaturated carboxylic acid or the salt thereof.

The following detailed description provides examples and are explanatory only of the invention. Accordingly, the following detailed description should not be considered to be restrictive. Additional features or variations thereof can be provided in addition to those set forth herein, such as for example, various feature combinations and sub-combinations of these described in the detailed description.

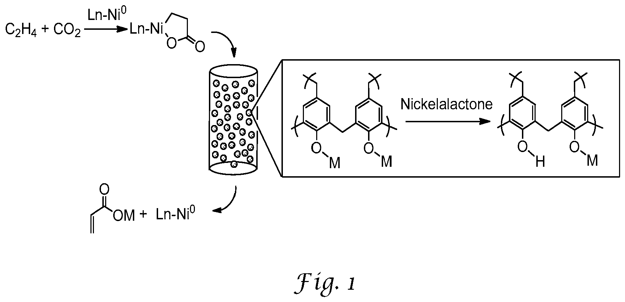

### BRIEF DESCRIPTION OF THE DRAWING

The FIGURE illustrates an embodiment or aspect of this disclosure, showing the use a porous crosslinked polyphenoxide resin stationary phase in a column configuration, in which formation of the acrylate coupling reaction of ethylene and CO₂ to form a metalalactone such as a nickelalactone in a mobile phase can be effected, and the resulting nickelalactone destabilized by the metallated crosslinked polyphenoxide resin stationary phase to form an acrylate product. The particular crosslinked polyphenoxide resin illustrated in this figure is merely representative of the numerous types of covalent linkages that typically exist in these types of resins.

### DETAILED DESCRIPTION OF THE DISCLOSURE

### Definitions

To define more clearly the terms used herein, the following definitions are provided. Unless otherwise indicated, the following definitions are applicable to this disclosure. If a term is used in this disclosure but is not specifically defined herein, the definition from the IUPAC Compendium of Chemical Terminology, 2nd Ed (1997) can be applied, as long as that definition does not conflict with any other disclosure or definition applied herein, or render indefinite or non-enabled any claim to which that definition is applied. To the extent that any definition or usage provided by any document referenced conflicts with the definition or usage provided herein, the definition or usage provided herein controls.

While compositions and methods are described in terms of "comprising" various components or steps, the compositions and methods can also "consist essentially of" or "consist of" the various components or steps, unless stated otherwise.

The terms "a," "an," and "the" are intended to include plural alternatives, e.g., at least one. For instance, the disclosure of "a porous crosslinked polyphenoxide resin," "a diluent," "a catalyst," is meant to encompass one, or mixtures or combinations of more than one, porous crosslinked polyphenoxide resin, diluent, catalyst unless otherwise specified.

The terms "including", "with", and "having", as used herein, are defined as comprising (i.e., open language), unless specified otherwise.

The term "hydrocarbon" refers to a compound containing only carbon and hydrogen. Other identifiers can be utilized to indicate the presence of particular groups in the hydrocarbon, for instance, a halogenated hydrocarbon indicates the presence of one or more halogen atoms replacing an equivalent number of hydrogen atoms in the hydrocarbon.

As used herein, the term "α,β-unsaturated carboxylic acid" and its derivatives refer to a carboxylic acid having a carbon atom of a carbon-carbon double bond attached to the carbonyl carbon atom (the carbon atom bearing the double bonded oxygen atom). Optionally, the α,β-unsaturated carboxylic acid can contain other functional groups, heteroatoms, or combinations thereof.

The terms "polyphenol", "polyaromatic", and "polyaryloxy" are generally used herein to describe a specific type of porous crosslinked polyphenol resin or polymer based upon the phenol-formaldehyde crosslinked resins and their analogs, in which the phenol or aromatic group and methylene moieties are part of an extended crosslinked network, rather than being solely pendant groups that are bonded to a polymeric backbone. Therefore, aromatic groups in the polymeric structure are hydroxylated, or hydroxymetallated in the anionic form, or otherwise functionalized with a group that will carry the negative charge in the porous crosslinked polyphenoxide resin, for example, thiolate, alkyl amide. Crosslinked networks that are prepared using various substituted phenols or polyhydroxyarene co-monomers also included in this definition. The term "phenolic resin" may be used to describe these materials as well. In their anionic form, the polyphenol resins are termed in a corresponding fashion as crosslinked polyphenoxide or polyaryloxide resins. If the context allows, the recitation of a polyphenol or polyaryloxy resin also encompasses the corresponding anionic (metallated) polyphenoxide or polyaryloxide resins.

The terms "polyhydroxyarene" or "polyhydroxidearene" are used herein to refer to a resin or polymer based upon the phenol-formaldehyde crosslinked resins and their analogs, in which the phenol-type monomer includes more than one hydroxyl group. Resorcinol (also termed, benzenediol or m-dihydroxybenzene) is a typical polyhydroxyarene, and in its anionic form may be referred to as resorcinoxide.

For any particular compound or group disclosed herein, any name or structure presented is intended to encompass all conformational isomers, regioisomers, stereoisomers, and mixtures thereof that can arise from a particular set of substituents, unless otherwise specified. The name or structure also encompasses all enantiomers, diastereomers, and other optical isomers (if there are any) whether in enantiomeric or racemic forms, as well as mixtures of stereoisomers, as would be recognized by a skilled artisan, unless otherwise specified. For example, a general reference to pentane includes n-pentane, 2-methyl-butane, and 2,2-dimethylpropane; and a general reference to a butyl group includes a n-butyl group, a sec-butyl group, an iso-butyl group, and a t-butyl group.

Various numerical ranges are disclosed herein. When Applicants disclose or claim a range of any type, Applicants' intent is to disclose or claim individually each possible number that such a range could reasonably encompass, including end points of the range as well as any sub-ranges and combinations of sub-ranges encompassed therein, unless otherwise specified. For example, by disclosing a temperature of from 70 °C to 80 °C, Applicant's intent is to recite individually 70 °C, 71 °C, 72 °C, 73 °C, 74 °C, 75 °C, 76 °C, 77 °C, 78 °C, 79 °C, and 80 °C, including any sub-ranges and combinations of sub-ranges encompassed therein, and these methods of describing such ranges are interchangeable. Moreover, all numerical end points of ranges disclosed herein are approximate, unless excluded by proviso. As a representative example, if Applicants state that one or more steps in the processes disclosed herein can be conducted at a temperature in a range from 10 °C to 75 °C, this range should be interpreted as encompassing temperatures in a range from "about" 10 °C to "about" 75 °C.

Values or ranges may be expressed herein as "about", from "about" one particular value, and/or to "about" another particular value. When such values or ranges are expressed, other embodiments disclosed include the specific value recited, from the one particular value, and/or to the other particular value. Similarly, when values are expressed as approximations, by use of the antecedent "about," it will be understood that the particular value forms another embodiment. It will be further understood that there are a number of values disclosed therein, and that each value is also herein disclosed as "about" that particular value in addition to the value itself. In another aspect, use of the term "about" means ±20% of the stated value, ±15% of the stated value, ±10% of the stated value, ±5% of the stated value, or ±3% of the stated value.

Applicants reserve the right to proviso out or exclude any individual members of any such group of values or ranges, including any sub-ranges or combinations of sub-ranges within the group, that can be claimed according to a range or in any similar manner, if for any reason Applicants choose to claim less than the full measure of the disclosure, for example, to account for a reference that Applicants can be unaware of at the time of the filing of the application. Further, Applicants reserve the right to proviso out or exclude any individual substituents, analogs, compounds, ligands, structures, or groups thereof, or any members of a claimed group, if for any reason Applicants choose to claim less than the full measure of the disclosure, for example, to account for a reference or prior disclosure that Applicants can be unaware of at the time of the filing of the application.

The term "substituted" when used to describe a group, for example, when referring to a substituted analog of a particular group, is intended to describe the compound or group wherein any non-hydrogen moiety formally replaces hydrogen in that group or compound, and is intended to be non-limiting. A compound or group can also be referred to herein as "unsubstituted" or by equivalent terms such as "non-substituted," which refers to the original group or compound. "Substituted" is intended to be non-limiting and include inorganic substituents or organic substituents as specified and as understood by one of ordinary skill in the art.

The terms "contact product," "contacting,", are used herein to describe compositions and methods wherein the components are contacted together in any order, in any manner, and for any length of time, unless specified otherwise. For example, the components can be contacted by blending or mixing. Further, unless otherwise specified, the contacting of any component can occur in the presence or absence of any other component of the compositions and methods described herein. Combining additional materials or components can be done by any suitable method. Further, the term "contact product" includes mixtures, blends, solutions, slurries, reaction products, or combinations thereof. Although "contact product" can, and often does, include reaction products, it is not required for the respective components to react with one another. Similarly, "contacting" two or more components can result in a reaction product or a reaction mixture. Consequently, depending upon the circumstances, a "contact product" can be a mixture, a reaction mixture, or a reaction product.

Although any methods and materials similar or equivalent to those described herein can be used in the practice or testing of the invention, the typical methods and materials are herein described.

Moreover, any headings that are employed herein are also not intended to be used to construe the scope of the claims or to limit the scope of the subject matter that is disclosed herein. Any use of the past tense to describe any example otherwise indicated as constructive or prophetic is not intended to reflect that the constructive or prophetic example has actually been carried out.

Those skilled in the art will readily appreciate that many modifications are possible in the exemplary embodiments disclosed herein without materially departing from the novel teachings and advantages according to this disclosure. Accordingly, all such modifications and equivalents are intended to be included within the scope of this disclosure as defined in the following claims. Therefore, it is to be understood that resort can be had to various other aspects, embodiments, modifications, and equivalents thereof which, after reading the description herein, may suggest themselves to one of ordinary skill in the art without departing from the scope of the appended claims.

The publications discussed throughout the text are provided solely for their disclosure prior to the filing date of the present application. Nothing herein is to be construed as an admission that the inventors are not entitled to antedate such disclosure by virtue of prior invention.

The present disclosure is directed generally to methods for forming α,β-unsaturated carboxylic acids, or salts thereof. An illustrative example of a suitable α,β-unsaturated carboxylic acid is acrylic acid.

According to one aspect, this disclosure provides for the formation of an α,β-unsaturated carboxylic acids and salts thereof from metalalactones and porous crosslinked polyphenoxide resins. One example of the α,β-unsaturated carboxylic acid salt formation from exemplary metalalactones and porous crosslinked polyphenoxide resins is illustrated in Scheme 1, which provides for a nickel catalytic coupling reaction between an olefin and CO₂ and formation of an acrylate. As explained herein, Scheme 1 is not limiting but is exemplary, and each reactant, catalyst, polymer, and product are provided for illustrative purposes.

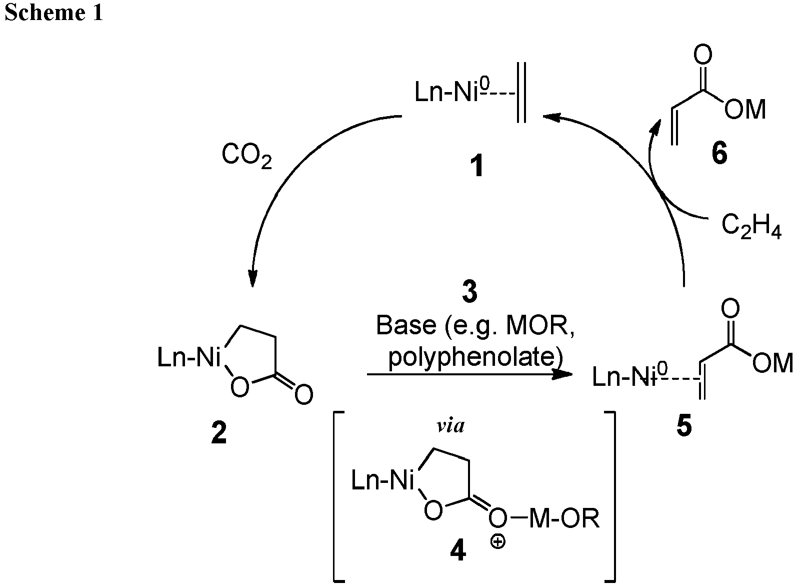

In Scheme 1, a transition metal catalyst as disclosed herein is illustrated generally by a nickel(0) catalyst at compound **1,** and the olefin disclosed herein, generally an α-olefin, is illustrated generally by ethylene. In the presence of the catalyst **1,** the olefin couples with CO₂ to form the metalalactone **2.** Metalalactone **2** is destabilized by its interaction with a heterogenized Lewis acid, i.e. a porous crosslinked metallated polyphenoxide resin **3** ("MOR" in Scheme 1). While not intending to be bound by theory, the metallated crosslinked polyphenoxide resin **3** is thought to interact with metalalactone **2** in some way, for example to form an adduct of some type, such as one illustrated as intermediate **4.** Reaction with the combined metallated crosslinked polyphenoxide resin **3** and metalalactone **2** (or intermediate of some type, represented generally as **4**) then proceeds to eliminate or release the metal acrylate **6,** for example from intermediate **4,** possibly by way of the nickel-acrylate adduct **5.** Ethylene displacement ultimately regenerates catalyst compound 1 and byproduct reacted polymer (here, crosslinked polyphenol resin, which is regenerated to the porous crosslinked polyphenoxide resin reactant, for example the metallated crosslinked polyphenoxide resin **3,** upon its reaction with a metal-stabilized base such as hydroxide or alkoxide. The participation of a solvent such as a polar solvent and/or base in the elimination or release of the metal acrylate **6,** is not fully understood at this time and may include direct participation in the mechanism or simply solvating an acrylate salt which is insoluble in the diluent. In other words, elimination of the metal acrylate from **4** occurs to regenerate catalyst compound **1** and byproduct reacted polymer (here, crosslinked polyphenol resin), which is regenerated to the porous crosslinked polyphenoxide resin reactant **3** upon its reaction with a metal-stabilized base (not shown in Scheme 1). In the presence of CO₂, the ethylene-stabilized adduct **1** is converted to metalalactone **2.**

One exemplary base illustrated in Scheme 1 is a hydroxide base, but a carbonate base, similar inorganic bases, and a wide range of other bases can be used, particularly metal-containing bases. Metal containing bases can include any basic inorganic metal compound or mixture of compounds that contain metal cations or cation sources, for example, alkali and alkaline earth metal compounds such as oxides, hydroxides, alkoxides, aryloxides, amides, alkyl amides, arylamides, and carbonates like calcium hydroxide. In an aspect, the reaction of Scheme 1 can be conducted using certain bases as disclosed, but if desired, other organic bases such as some alkoxide, aryloxide, amide, alkyl amide, arylamide bases, can be excluded. Typically, the inorganic bases such as alkali metal hydroxides have been found to work well.

An aspect of this disclosure is the high porosity and high density of associated metal (e.g. sodium) sites that can be achieved with the polyphenoxide resin is prepared and crosslinked using a templating process. The present invention provides a process for forming a porous crosslinked polyphenoxide resin, the process comprising:
a) in the presence of a basic particulate template, contacting at least one phenol compound, formaldehyde, and an aqueous base under polymerization conditions sufficient to form a templated crosslinked polyphenol resin comprising a crosslinked polyphenol resin in contact with the basic particulate template;
b) contacting the templated crosslinked polyphenol resin with an aqueous acid under pore forming conditions sufficient to remove the basic particulate template and form a porous crosslinked polyphenol resin; and
c) contacting the porous crosslinked polyphenol resin with a metal-containing base to form a promoter comprising a porous crosslinked polyphenoxide resin comprising associated metal cations.
When the phenol compound, formaldehyde, and the aqueous base are contacted in the presence of a basic particulate template, the polymerization conditions that can be applied include selecting the order of addition of the reactants, selecting the particular base and its concentration, adjusting the temperature and the temperature gradient(s), adjusting the reaction times, selecting the diluent and any (co)diluent, selecting other components, including for example, other components to achieve emulsion polymerization. When the templated crosslinked polyphenol resin is contacted with an aqueous acid, the pore forming conditions sufficient to remove the basic particulate template and form a porous crosslinked polyphenol resin can include selecting the specific aqueous acid, adjusting the aqueous acid concentration or pH, adjusting the temperature and any temperature gradient(s), adjusting the reaction times, selecting the diluent and any (co)diluent, and any subsequent wash or processing steps. These are examples of the polymerization conditions and the pore forming conditions that can be used in these respective processes and are not intended to be exhaustive or limiting.

When prepared in this fashion, the porous crosslinked polyphenoxide resin can be mesoporous, having an average pore diameter from about 2 nm to about 50 nm. Alternatively, the porous crosslinked polyphenoxide resin can be macroporous, having an average pore diameter greater than about 50 nm. In another aspect, the porous crosslinked polyphenoxide resin can have an average pore diameter from about 50 nm to about 250 nm. These pore diameters can be adjusted by, for example, the size of the basic particulate template used in preparing the crosslinked resin, by the extent of crosslinking reaction when the phenol compound and formaldehyde are contacted with varying amounts of aqueous base and/or reaction times and polymerization conditions. Surface area, pore diameter, and pore volume were measured by Brunauer, Emmett and Teller (BET) technique with nitrogen gas used as the probe.

Generally, the porous crosslinked polyphenoxide resin and associated cations used in the processes disclosed herein can comprise (or consist essentially of, or consist of) an insoluble porous crosslinked polyphenoxide resin, a solvent-swellable porous crosslinked polyphenoxide resin, or a combination thereof. It is further contemplated that mixtures or combinations of two or more porous crosslinked polyphenoxide resins can be employed in certain aspects of the disclosure. Therefore, the "porous crosslinked polyphenoxide resin" is a polymeric material which comprises a multiply-charged polyanion, together with an equivalent amount of counter cations, and is used generally to refer to both insoluble materials and solvent-swellable materials.

In an aspect, the porous crosslinked polyphenoxide resin (and associated cations) can be used in the absence of an alkoxide or aryloxide base. Further, the reactions and processes disclosed herein can be conducted in the absence of an alkoxide, an aryloxide, an alkylamide, an arylamide, and/or substituted analogs thereof. That is, additional bases with their associated counter ions are not required to effect the processes disclosed herein.

According to an aspect, the porous crosslinked polyphenoxide resin and associated cations used in the processes can be used in the absence of a solid support. That is the porous crosslinked polyphenoxide resin can be used is its natural polymeric form without being bonded to or supported on any insoluble support, such as an inorganic oxide or mixed oxide material.

Accordingly, the terms crosslinked polyphenol resin and crosslinked polyphenoxide resin are used generally to include such crosslinked polyphenol or polyphenoxide resins as a phenol-formaldehyde resin, a polyhydroxyarene-formaldehyde resin (such as a resorcinol-formaldehyde resin), a polyhydroxyarene- and fluorophenol-formaldehyde resin (such as a resorcinol- and 2-fluorophenol-formaldehyde resin), or combinations thereof. In their deprotonated form, these porous crosslinked polyphenoxide resins comprise metal cations associated with the phenoxide-formaldehyde resin, a polyhydroxidearene-formaldehyde resin (such as a resorcinoxide-formaldehyde resin), a polyhydroxidearene- and fluorophenoxide-formaldehyde resin (such as a resorcinoxide- and 2-fluorophenoxide-formaldehyde resin), including combinations thereof.

Thus, one aspect of the disclosed process provides for using a porous crosslinked polyphenoxide resin that comprises, consists essentially of, or consists of a phenoxide-formaldehyde resin, a polyhydroxidearene-formaldehyde resin (such as a resorcinoxide-formaldehyde resin), a polyhydroxidearene- and fluorophenoxide-formaldehyde resin (such as a resorcinoxide- and 2-fluorophenoxide-formaldehyde resin), or combinations thereof. For example, these resins include a phenoxide-formaldehyde resin, a resorcinoxide-formaldehyde resin, a resorcinoxide- and 2-fluorophenoxide-formaldehyde resin, or any combinations thereof.

In an aspect, a variety of substituted phenols can be used to prepare the phenol-formaldehyde type of crosslinked resins. Examples include phenols that are substituted with at least one electron withdrawing group. When multiple electron withdrawing groups are present, the electron withdrawing groups can be the same or can be different. For example, the phenol can be substituted with fluorine in one or more than one position. The fluorine can be *ortho* to the phenol hydroxyl group or can be at other positions, and the phenol can be multiply substituted with an electron withdrawing group such as fluorine substituents. Bulky *ortho* substituents such as an *ortho-t*-butyl group can be used (that is, ortho-t-butyl phenol as a reactant), which can also provide the benefit of largely preventing carbonate formation.

These polymers that generally fall under the phenol-formaldehyde type of crosslinked resins also may be referred to as polyaromatic resins, and these polyelectrolyte core structures generally constitute part of the polymer backbone. Substituted variations are included in this disclosure, and use of the term porous crosslinked polyphenol or polyphenoxide resin includes, for example, those polyphenol or polyphenoxide resins that are substituted with electron-withdrawing groups or electron-donating groups or even combinations thereof.

Porous crosslinked polyphenoxide resins such as those used herein include associated cations, particularly associated metal cations, including Lewis acidic metal cations and cations with low Lewis acidity. According to an aspect, the associated metal cations can be an alkali metal, an alkaline earth metal, or any combination thereof. Typical associated metal cations can be, can comprise, or can be selected from lithium, sodium, potassium, magnesium, calcium, strontium, barium, aluminum, or zinc. Generally, sodium or potassium associated metal cations have been found to work well. Therefore, cations with a range of Lewis acidities in the particular solvent can be useful according to this disclosure.

The templating process that provides the high porosity and high density of associated metal (e.g. sodium) sites can use a basic particulate template, that has a solubility in water of less than about 0.25 g/L at 25 °C. The basic particulate template also can have a solubility in water of less than about 0.10 g/L at 25 °C. In an aspect, the basic particulate template, can have a solubility in water (all measured at 25 °C) of less than about 0.001 g/L, about 0.001 g/L, about 0.002 g/L, about 0.005 g/L, about 0.01 g/L, about 0.02 g/L, about 0.03 g/L, about 0.04 g/L, about 0.05 g/L, about 0.06 g/L, about 0.07 g/L, about 0.08 g/L, about 0.09 g/L, about 0.10 g/L, about 0.11 g/L, about 0.12 g/L, about 0.13 g/L, about 0.14 g/L, about 0.15 g/L, about 0.16 g/L, about 0.17 g/L, about 0.18 g/L, about 0.19 g/L, about 0.20 g/L, about 0.21 g/L, about 0.22 g/L, about 0.23 g/L, about 0.24 g/L, about 0.25 g/L, about 0.26 g/L, about 0.27 g/L, about 0.28 g/L, about 0.29 g/L, about 0.30 g/L, or greater than about 0.30 g/L (up to, for example, about 0.50 g/L), including any ranges or combination of ranges between any of these solubilities. For example, the basic particulate template also can have a solubility in water of from about 0.005 g/L to about 0.50 g/L, from about 0.5 g/L to about 0.50 g/L, from about 0.01 g/L to about 0.25 g/L, or about 0.05 g/L to about 0.20 g/L at 25 °C.

The size of the basic particulate template also can vary, for example, the basic particulate template can have an average or median particle size from about 0.1 µm (micrometers) to about 50 µm, or can have an average or median particle size from about 10 µm (micrometers) to about 25 µm. In an aspect, the average or median particle size is measured by either dynamic light scattering tests or by a laser diffraction technique. In this aspect, the basic particulate template can have an average or median particle size of less than about 0.1 µm, about 0.2 µm, about 0.5 µm, about 1 µm, about 2 µm, about 3 µm, about 4 µm, about 5 µm, about 6 µm, about 7 µm, about 8 µm, about 9 µm, about 10 µm, about 11 µm, about 12 µm, about 15 µm, about 20 µm, about 25 µm, about 30 µm, about 35 µm, about 40 µm, about 45 µm, about 50 µm, about 55 µm, about 60 µm, about 65 µm, about 70 µm, about 75 µm, about 80 µm, about 85 µm, about 90 µm, about 95 µm, about 100 µm, about 125 µm, about 150 µm, about 175 µm, about 200 µm, about 250 µm, about 300 µm, about 350 µm, about 400 µm, about 450 µm, or about 500 µm, or greater than about 500 µm (up to, for example, about 750 µm), including any ranges or combination of ranges between any of these sizes. In a further aspect, nanometer-scale calcium carbonate can be prepared and used as a template to form a nanometer-scale porous crosslinked polyphenoxide resins. For example, nanometer-scale calcium carbonate can be prepared from calcium chloride and carbonic acid to control particulate size and morphology.

According to an aspect, the basic particulate template can comprise an alkaline earth metal carbonate, phosphate, monohydrogen phosphate, or dihydrogen phosphate, or combinations thereof. The basic particulate template generally has low solubility in water, and it can be reacted with aqueous acid to form soluble salts and other species, that allow for the formation of the porous structure of the crosslinked polyphenoxide resin. For example, the basic particulate template can comprise, consist essentially of, or consist of magnesium carbonate, calcium carbonate, strontium carbonate, tribasic calcium phosphate, calcium monohydrogen phosphate, or calcium dihydrogen phosphate, or combinations thereof. The basic particulate template comprises or can be selected from magnesium carbonate or calcium carbonate. Calcium carbonate is a useful basic templating material, and samples can have, for example, an average particle size from about 2 µm (micrometers) to about 200 µm, about 2 µm to about 100 µm, or about 2 µm to about 50 µm.

To form the templated polyphenol resin, at least one phenol compound, formaldehyde, and an aqueous base are contacted with the basic particulate template under polymerization conditions sufficient to form a templated crosslinked polyphenol resin, which comprises the crosslinked polyphenol resin in contact with the basic particulate template. The aqueous base can comprise or be selected from any suitable aqueous base or any aqueous base disclosed herein, for example, an alkaline metal hydroxide such as NaOH or KOH.

Once templated in this fashion, the templated crosslinked polyphenol resin is contacted with an aqueous acid under pore forming conditions sufficient to remove the basic particulate template and form a porous crosslinked polyphenol resin. The aqueous acid can comprise or can be selected from any suitable aqueous acid or any aqueous acid disclosed herein, for example, the aqueous acid can be a hydrohalic acid such as HCl(aq) or HBr(aq), but acids like aqueous nitric acid or sulfuric acid can be used. Strong organic acids can even be used in this fashion, for example, p-toluenesulfonic acid or methanesulfonic acid can be used for this process.

As noted, advantages of using treated phenol-formaldehyde resins include their insolubility, which allows the use of a range of solvents with these materials, and their relatively high phenol concentration that can be functionalized using a metal base such as an alkali metal hydroxide. An early version of the thermosetting phenol-formaldehyde resins formed from the condensation reaction of phenol with formaldehyde is Bakelite^{™}, and various phenol-formaldehyde resins used herein may be referred to generically as "Bakelite" resins. In the context of this disclosure, the use of terms such as Bakelite or general terms such as phenol-formaldehyde resins contemplates that these materials will be treated with a metal-containing base or a metal cation source such as sodium hydroxide prior to their use in the processes disclosed.

In addition, other useful porous crosslinked polyphenol resins include substituted phenol-formaldehyde resins that are also generally crosslinked into insoluble resins. These resins can be formed from the condensation reaction of one or more of phenol, a polyhydroxyarene such as resorcinol (also, benzenediol or m-dihydroxybenzene), and/or their substituted analogs with formaldehyde. Therefore, these materials include resins made with more than one phenol as co-monomer. Treatment with bases such as NaOH or KOH also provides a ready method of functionalizing the polyaromatic polymers for the reactivity described herein.

In one example, a resin can be prepared using the monomer combination of resorcinol (m-dihydroxybenzene) and fluorophenol monomers with formaldehyde, and sodium-treated to generate the porous crosslinked polyphenol resin. While not intending to be theory bound, the meta-dihydroxybenzene is believed to add additional ion chelation functionality to the resin. Subsequent base (e.g. sodium hydroxide) treatment can be used to generate the porous crosslinked polyphenoxide that is a polyhydroxidearene resin. Such adjustments can provide flexibility for tailoring the reaction according to the specific olefin to be coupled with CO₂, the reaction rate, the catalytic turnover, as well as additional reaction parameters and combinations of reaction parameters.

In other aspects and embodiments in which polymer support variations are used and/or in which the porous crosslinked polyphenoxide resin itself, after the templating synthesis, is used without a support, the porous crosslinked polyphenoxide resin embodiments can have any suitable surface area, pore volume, and particle size, as would be recognized as acceptable by those of skill in the art. For instance, the porous crosslinked polyphenoxide resin can have a pore volume in a range from 0.1 mL/g to 25 mL/g, from 0.5 mL/g to 10 mL/g, or alternatively, from 0.5 mL/g to 2.5 mL/g. In a further aspect, the porous crosslinked polyphenoxide can have a pore volume from 1 mL/g to 8 mL/g, or alternatively from 2 mL/g to 15 mL/g. Additionally, or alternatively, the porous crosslinked polyphenoxide resin can have a BET surface area in a range from 10 to 1,000 m²/g; alternatively, from 100 to 750 m²/g; or alternatively, from 100 to 500 m²/g or alternatively from 30 to 200 m²/g. In a further aspect, the porous crosslinked polyphenoxide resin can have a surface area of from 100 to 400 m²/g, from 200 to 450 m²/g, or from 150 to 350 m²/g. The average particle size of the porous crosslinked polyphenoxide resin can vary greatly depending upon the process specifics, however, average particle sizes in the range of from 2 to 500 µm, from 10 to 250 µm, or from 15 to 100 µm, are often employed. IN one aspect, the average or median particle size of the porous crosslinked polyphenoxide resin can mirror the average or median sizes recited for the basic particulate template.

The present disclosure also provides for various modifications of the polymeric anionic stationary phase (porous crosslinked polyphenoxide resins), for example, in a column or other suitable solid state configuration. Further various modifications of the polymeric anionic stationary phase (porous crosslinked polyphenoxide resins), for example, in a column or other suitable solid state configuration are useful in the processes disclosed herein. For example, acid-base reactions that generate the porous crosslinked polyphenoxide resin from the reacted polymer can be effected using a wide range of metal bases, including alkali and alkaline hydroxides, alkoxides, aryloxides, amides, alkyl or aryl amides, such that an assortment of electrophiles can be used in nickelalactone destabilization.

According to an aspect, disclosed herein is a porous crosslinked polyphenol resin, the resin comprising a phenol-formaldehyde resin, a polyhydroxyarene-formaldehyde resin, a polyhydroxyarene- and fluorophenol-formaldehyde resin, or any combination thereof, and having an average particle size from about 2 µm (micrometers) to about 50 µm and an average pore diameter from about 2 nm (nanometers) to about 250 nm. In a further aspect, there is provided a porous crosslinked polyphenoxide resin, the resin comprising a phenoxide-formaldehyde resin, a polyhydroxidearene-formaldehyde resin, a polyhydroxidearene- and fluorophenoxide-formaldehyde resin, or any combination thereof; and associated metal cations comprising lithium, sodium, potassium, magnesium, calcium, strontium, barium, aluminum, or zinc; wherein the porous crosslinked polyphenoxide resin has an average particle size from about 2 µm (micrometers) to about 50 µm and an average pore diameter from about 2 nm (nanometers) to about 250 nm.

Referring again to Scheme 1, the disclosed processes can further include the step of reacting the byproduct reacted polymer, such as a crosslinked polyphenol resin, with a base. For example a base, which are also termed a regenerative base, can be used to regenerate the crosslinked polyphenol resin byproduct to the porous crosslinked polyphenoxide resin reactant. The regenerative base can comprise a metal ion or a metal ion source, for example a metal-stabilized base such as metal hydroxide or metal alkoxide can be used. Thus, in the example of Scheme 1, the porous crosslinked polyphenoxide resin can be a metallated crosslinked polyphenoxide resin, which is formed upon the reaction of the reacted polymer, for example crosslinked polyphenol resin, with a base such as a metal-containing base. For example, the metal in a metal-containing base can be a metal of Groups 1, 2, 12 or 13, such as lithium, sodium, potassium, rubidium, cesium, magnesium, calcium, zinc, aluminum or gallium.

The step of regenerating the porous crosslinked polyphenoxide resin can be effected by contacting the porous crosslinked polyphenol resin with a regenerative base comprising a metal cation following the formation of the α,β-unsaturatedcarboxylic acid or a salt thereof. A wide range of bases can be used for this regeneration step. For example, the regenerative base can be or can comprise metal-containing bases which can include any reactive inorganic basic metal compound or mixture of compounds that contain metal cations or cation sources, for example, alkali and alkaline earth metal compounds such as oxides, hydroxides, alkoxides, aryloxides, amides, alkyl amides, arylamides, and carbonates. Suitable bases include or comprise, for example, carbonates (e.g., Na₂CO₃, Cs₂CO₃, MgCO₃), hydroxides (e.g., Mg(OH)₂, Ca(OH)₂, NaOH, KOH), alkoxides (e.g., Al(O*ⁱ*Pr)₃, Na(O*^{t}*Bu), Mg(OEt)₂), aryloxides (e.g. Na(OC₆H₅), sodium phenoxide). In an aspect, certain porous crosslinked polyphenol resins with particularly acidic phenolic groups can be regenerated to the porous crosslinked polyphenoxide resin reactant upon its reaction with only a metal-containing salt such as sodium chloride. Such resins can have electron-withdrawing substituents situated *ortho* or *para* to the phenol hydroxyl group, such that the anionic form can readily form and only a metal-containing salt (or "metal salt") such as sodium chloride is required to regenerate polyphenoxide resin. Typically, this regeneration step further comprising or is followed by the step of washing the porous crosslinked polyphenoxide resin with a solvent or the diluent.

According to an aspect, the regenerative base can be or can comprise a nucleophilic base, for example a metal hydroxide or metal alkoxide. While the regenerative base can comprise a non-nucleophilic base, the processes disclosed herein works well in the absence of non-nucleophilic bases, for example, in the absence of an alkali metal hydride or an alkaline earth metal hydride, an alkali metal or alkaline earth metal dialkylamides and diarylamides, an alkali metal or alkaline earth metal hexalkyldisilazane, and an alkali metal or alkaline earth metal dialkylphosphides and diarylphosphides. Therefore, in a particular aspect, the regenerating process can be carried out in the absence of a non-nucleophilic base, such as in the absence of a metal hydride.

Typically, the inorganic bases such as alkali metal hydroxides or alkali metal alkoxides have been found to work the best. However, in one aspect, the reaction of Scheme 1 can be conducted using some bases but in the absence of certain other organic bases such as an alkoxide, aryloxide, amide, alkyl amide, arylamide. In another aspect, the porous crosslinked polyphenoxide resin (and associated cations) can be used and regenerated in the absence of an alkoxide or aryloxide. Further, the reactions and processes disclosed herein can be conducted in the absence of an alkoxide, an aryloxide, an alkylamide, an arylamide, an amine, a hydride, a phosphazene, and/or substituted analogs thereof. For example, the processes disclosed herein can be conducted in the absence of sodium hydride, an aryloxide salt (such as a sodium aryloxide), an alkoxide salt (such as a sodium tert-butoxide), and/or a phosphazene.

The processes disclosed herein typically are conducted in the presence of a diluent. Mixtures and combinations of diluents can be utilized in these processes. The diluent can comprise, consist essentially of, or consist of, any suitable solvent or any solvent disclosed herein, unless otherwise specified. For example, the diluent can comprise, consist essentially of, or consist of a non-protic solvent, a protic solvent, a non-coordinating solvent, or a coordinating solvent. For instance, in accordance with one aspect of this disclosure, the diluent can comprise a non-protic solvent. Representative and non-limiting examples of non-protic solvents can include tetrahydrofuran (THF), 2,5-Me₂THF, acetone, toluene, chlorobenzene, pyridine, acetonitrile, carbon dioxide, olefin, as well as combinations thereof. In accordance with another aspect, the diluent can comprise a weakly coordinating or non-coordinating solvent. Representative and non-limiting examples of weakly coordinating or non-coordinating solvents can include toluene, chlorobenzene, paraffins, halogenated paraffins, as well as combinations thereof.

In accordance with yet another aspect, the diluent can comprise a carbonyl-containing solvent, for instance, ketones, esters, amides, as well as combinations thereof. Representative and non-limiting examples of carbonyl-containing solvents can include acetone, ethyl methyl ketone, ethyl acetate, propyl acetate, butyl acetate, isobutyl isobutyrate, methyl lactate, ethyl lactate, N,N-dimethylformamide, as well as combinations thereof. In still another aspect, the diluent can comprise THF, 2,5-Me₂THF, methanol, acetone, toluene, chlorobenzene, pyridine, acetonitrile, anisole, or a combination thereof; alternatively, THF; alternatively, 2,5-Me₂THF; alternatively, methanol; alternatively, acetone; alternatively, toluene; alternatively, chlorobenzene; or alternatively, pyridine.

In an aspect, the diluent can comprise (or consist essentially of, or consist of) an aromatic hydrocarbon solvent. Non-limiting examples of suitable aromatic hydrocarbon solvents that can be utilized singly or in any combination include benzene, toluene, xylene (inclusive of ortho-xylene, meta-xylene, para-xylene, or mixtures thereof), and ethylbenzene, or combinations thereof; alternatively, benzene; alternatively, toluene; alternatively, xylene; or alternatively, ethylbenzene.

In an aspect, the diluent can comprise (or consist essentially of, or consist of) a halogenated aromatic hydrocarbon solvent. Non-limiting examples of suitable halogenated aromatic hydrocarbon solvents that can be utilized singly or in any combination include chlorobenzene, dichlorobenzene, and combinations thereof; alternatively, chlorobenzene; or alternatively, dichlorobenzene.

In an aspect, the diluent can comprise (or consist essentially of, or consist of) an ether solvent. Non-limiting examples of suitable ether solvents that can be utilized singly or in any combination include dimethyl ether, diethyl ether, diisopropyl ether, di-n-propyl ether, di-n-butyl ether, diphenyl ether, methyl ethyl ether, methyl t-butyl ether, dihydrofuran, tetrahydrofuran (THF), 2,5-Me₂THF, 1,2-dimethoxyethane, 1,4-dioxane, anisole, and combinations thereof; alternatively, diethyl ether, dibutyl ether, THF, 2,5-Me₂THF, 1,2-dimethoxyethane, 1,4-dioxane, and combinations thereof; alternatively, THF; or alternatively, diethyl ether.

In a further aspect, any of these aforementioned diluents can be excluded from the diluent or diluent mixture. For example, the diluent can be absent a phenol or a substituted phenol, an alcohol or a substituted alcohol, an amine or a substituted amine, water, an ether, an aliphatic hydrocarbon solvent, an aromatic hydrocarbon solvent, an aldehyde or ketone, an ester or amide, and/or absent a halogenated aromatic hydrocarbon, or any substituted analogs of these diluents halogenated analogs, including any of the aforementioned diluents. Therefore, Applicant reserves the right to exclude any of the diluents provided herein.

In all aspects and embodiments disclosed herein, the diluent can include or comprise carbon dioxide, olefin, or combinations thereof. At least a portion of the diluent can comprise the α,β-unsaturated carboxylic acid or the salt thereof, formed in the process.

In this disclosure, the term transition metal precursor, transition metal compound, transition metal catalyst, transition metal precursor compound, carboxylation catalyst, transition metal precursor complex, transition metal-ligand complex, and similar terms refer to a chemical compound that serves as the precursor to the metalalactone, prior to the coupling of the olefin and carbon dioxide at the metal center of the transition metal precursor compound. Therefore, the metal of the transition metal precursor compound and the metal of the metalalactone are the same. In some aspects, some of the ligands of the transition metal precursor compound carry over and are retained by the metalalactone following the coupling reaction. In other aspects, the transition metal precursor compound loses its existing ligands, referred to herein as first ligands, in presence of additional ligands such as chelating ligands, referred to herein as second ligands, as the metalalactone is formed. Therefore, the metalalactone generally incorporates the second (added) ligand(s), though in some aspects, the metalalactone can comprise the first ligand(s) that were bound in the transition metal precursor compound.

According to an aspect, the transition metal catalyst or compound used in the processes can be used without being immobilized on a solid support. That is the transition metal catalyst can be used is its usual form which is soluble in most useful solvents, without being bonded to or supported on any insoluble support, such as an inorganic oxide or mixed oxide material.

A prototypical example of a transition metal precursor compound that loses its initial ligands in the coupling reaction in the presence of a second (added) ligand, wherein the metalalactone incorporates the second (added) ligand(s), is contacting Ni(COD)₂ (COD is 1,5-cyclooctadiene) with a diphosphine ligand such as 1,2-bis(dicyclohexylphosphino)ethane in a diluent in the presence of ethylene and CO₂ to form a nickelalactone with a coordinated 1,2-bis(dicyclohexylphosphino)ethane bidentate ligand.

According to an aspect, any of the metalalactone ligand (that is, any ligand of the metalalactone compound other than the metalalactone moiety), the first ligand, or the second ligand can be any suitable neutral electron donor group and/or Lewis base, or any neutral electron donor group and/or Lewis base disclosed herein. For example, any of the metalalactone ligand, the first ligand, or the second ligand can be a bidentate ligand. Any of the metalalactone ligand, the first ligand, or the second ligand can comprise at least one of a nitrogen, phosphorus, sulfur, or oxygen heteroatom. For example, any of the metalalactone ligand, the first ligand, or the second ligand comprises or is selected from a diphosphine ligand, a diamine ligand, a diene ligand, a diether ligand, or dithioether ligand.

The present inventions also provides a process for forming an α,β-unsaturated carboxylic acid or a salt thereof, comprising:
a) contacting in any order
   1) a transition metal precursor compound comprising at least one first ligand;
   2) optionally, at least one second ligand;
   3) an olefin;
   4) carbon dioxide (CO₂);
   5) a diluent; and
   6) a promoter comprising a porous crosslinked polyphenoxide resin comprising associated metal cations to provide a reaction mixture; and
b) applying reaction conditions to the reaction mixture suitable to form the α,β-unsaturated carboxylic acid or the salt thereof.

Generally, the processes disclosed herein employ a metalalactone or a transition metal precursor compound or complex. The transition metal of the metalalactone, or of the transition metal precursor compound, can be a Group 3 to Group 8 transition metal or, alternatively, a Group 8 to Group 11 transition metal. In one aspect, for instance, the transition metal can be Fe, Co, Ni, Cu, Ru, Rh, Pd, Ag, Ir, Pt, or Au, while in another aspect, the transition metal can be Fe, Ni, or Rh. Alternatively, the transition metal can be Fe; alternatively, the transition metal can be Co; alternatively, the transition metal can be Ni; alternatively, the transition metal can be Cu; alternatively, the transition metal can be Ru; alternatively, the transition metal can be Rh; alternatively, the transition metal can be Pd; alternatively, the transition metal can be Ag; alternatively, the transition metal can be Ir; alternatively, the transition metal can be Pt; or alternatively, the transition metal can Au.

In particular aspects contemplated herein, the transition metal can be Ni. Hence, the metalalactone can be a nickelalactone and the transition metal precursor compound can be a Ni-ligand complex in these aspects.

The ligand of the metalalactone and/or of the transition metal precursor compound, can be any suitable neutral electron donor group and/or Lewis base. For instance, the suitable neutral ligands can include sigma-donor solvents that contain a coordinating atom (or atoms) that can coordinate to the transition metal of the metalalactone (or of the transition metal precursor compound). Examples of suitable coordinating atoms in the ligands can include O, N, S, and P, or combinations of these atoms. In some aspects consistent with this disclosure, the ligand can be a bidentate ligand.

In an aspect, the ligand used to form the metalalactone and/or the transition metal precursor compound can be an ether, an organic carbonyl, a thioether, an amine, a nitrile, or a phosphine. In another aspect, the ligand used to form the metalalactone or the transition metal precursor compound can be an acyclic ether, a cyclic ether, an acyclic organic carbonyl, a cyclic organic carbonyl, an acyclic thioether, a cyclic thioether, a nitrile, an acyclic amine, a cyclic amine, an acyclic phosphine, or a cyclic phosphine.

Suitable ethers can include dimethyl ether, diethyl ether, dipropyl ether, dibutyl ether, methyl ethyl ether, methyl propyl ether, methyl butyl ether, diphenyl ether, ditolyl ether, tetrahydrofuran, 2-methyltetrahydrofuran, 2,5-dimethyltetrahydrofuran, 2,3-dihydrofuran, 2,5-dihydrofuran, furan, benzofuran, isobenzofuran, dibenzofuran, tetrahydropyran, 3,4-dihydro-2H-pyran, 3,6-dihydro-2H-pyran, 2H-pyran, 4H-pyran, 1,3-dioxane, 1,4-dioxane, morpholine, including substituted derivatives thereof.

Suitable organic carbonyls can include ketones, aldehydes, esters, and amides, either alone or in combination, and illustrative examples can include acetone, acetophenone, benzophenone, N,N-dimethylformamide, N,N-dimethylacetamide, methyl acetate, ethyl acetate, including substituted derivatives thereof.

Suitable thioethers can include dimethyl thioether, diethyl thioether, dipropyl thioether, dibutyl thioether, methyl ethyl thioether, methyl propyl thioether, methyl butyl thioether, diphenyl thioether, ditolyl thioether, thiophene, benzothiophene, tetrahydrothiophene, thiane, including substituted derivatives thereof.

Suitable nitriles can include acetonitrile, propionitrile, butyronitrile, benzonitrile, 4-methylbenzonitrile, including substituted derivatives thereof.

Suitable amines can include methyl amine, ethyl amine, propyl amine, butyl amine, dimethyl amine, diethyl amine, dipropyl amine, dibutyl amine, trimethyl amine, triethyl amine, tripropyl amine, tributyl amine, aniline, diphenylamine, triphenylamine, tolylamine, xylylamine, ditolylamine, pyridine, quinoline, pyrrole, indole, 2-methylpyridine, 3-methylpyridine, 4-methylpyridine, 2,5-dimethylpyrrole, 2,5-diethylpyrrole, 2,5-dipropylpyrrole, 2,5-dibutylpyrrole, 2,4-dimethylpyrrole, 2,4-diethylpyrrole, 2,4-dipropylpyrrole, 2,4-dibutylpyrrole, 3,4-dimethylpyrrole, 3,4-diethylpyrrole, 3,4-dipropylpyrrole, 3,4-dibutylpyrrole, 2-methylpyrrole, 2-ethylpyrrole, 2-propylpyrrole, 2-butylpyrrole, 3-methylpyrrole, 3-ethylpyrrole, 3-propylpyrrole, 3-butylpyrrole, 3-ethyl-2,4-dimethylpyrrole, 2,3,4,5-tetramethylpyrrole, 2,3,4,5-tetraethylpyrrole, 2,2'-bipyridine, 1,8-Diazabicyclo[5.4.0]undec-7-ene, di(2-pyridyl)dimethylsilane, N,N,N',N'-tetramethylethylenediamine, 1,10-phenanthroline, 2,9-dimethyl-1,10-phenanthroline, glyoxal-bis(mesityl)-1,2-diimine, including substituted derivatives thereof. Suitable amines can be primary amines, secondary amines, or tertiary amines.

Suitable phosphines and other phosphorus compounds can include, trimethylphosphine, triethylphosphine, tripropylphosphine, tributylphosphine, phenylphosphine, tolylphosphine, diphenylphosphine, ditolylphosphine, triphenylphosphine, tritolylphosphine, methyldiphenylphosphine, dimethylphenylphosphine, ethyldiphenylphosphine, diethylphenylphosphine, tricyclohexylphosphine, trimethyl phosphite, triethyl phosphite, tripropyl phosphite, triisopropyl phosphite, tributyl phosphite and tricyclohexyl phosphite, 2-(di-t-butylphosphino)biphenyl, 2-di-t-butylphosphino-1,1'-binaphthyl, 2-(di-t-butylphosphino)-3,6-dimethoxy-2',4',6'-tri-i-propyl-1,1'-biphenyl, 2-di-t-butylphosphino-2'-methylbiphenyl, 2-(di-t-butylphosphinomethyl)pyridine, 2-di-t-butylphosphino-2',4',6'-tri-i-propyl-1,1'-biphenyl, 2-(dicyclohexylphosphino)biphenyl, (S)-(+)-(3,5-dioxa-4-phospha-cyclohepta[2,1-a;3,4-a']dinaphthalen-4-yl)dimethylamine, 2-(diphenylphosphino)-2'-methoxy-1,1'-binaphthyl, 1,2,3,4,5-pentaphenyl-1'-(di-t-butylphosphino)ferrocene, 2,2'-bis(diphenylphosphino)-1,1'-binaphthyl (BINAP), 1,2-bis(dimethylphosphino)ethane, 1,2-bis(diethylphosphino)ethane, 1,2-bis(dipropylphosphino)-ethane, 1,2-bis(diisopropylphosphino)ethane, 1,2-bis(dibutyl-phosphino)ethane, 1,2-bis(di-t-butyl-phosphino)ethane, 1,2-bis(dicyclohexylphosphino)ethane, 1,3-bis(dicyclohexylphosphino)propane, 1,3-bis(diisopropylphosphino)propane, 1,3-bis(diphenylphosphino)propane, 1,3-bis(di-t-butylphosphino)propane, 1,4-bis(diisopropylphosphino)butane, 1,4-bis(diphenylphosphino)butane, 2,2'-bis[bis(3,5-dimethylphenyl)phosphino]-4,4',6,6'-tetramethoxybiphenyl, 2,6-bis(di-t-butylphosphinomethyl)pyridine, 2,2'-bis(dicyclohexylphosphino)-1,1'-biphenyl, bis(2-dicyclohexylphosphinophenyl)ether, 5,5'-bis(diphenylphosphino)-4,4'-bi-1,3-benzodioxole, 2-t-butylphosphinomethylpyridine, bis(diphenylphosphino)ferrocene, bis(diphenylphosphino)methane, bis(dicyclohexylphosphino)methane, bis(di-t-butylphosphino)methane, including substituted derivatives thereof.

In other aspects, the ligand used to form the metalalactone or the transition metal precursor compound can be a carbene, for example, a N-heterocyclic carbene (NHC) compound. Representative and non-limiting examples of suitable N-heterocyclic carbene (NHC) materials include the following: 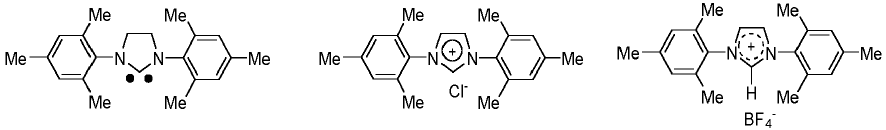 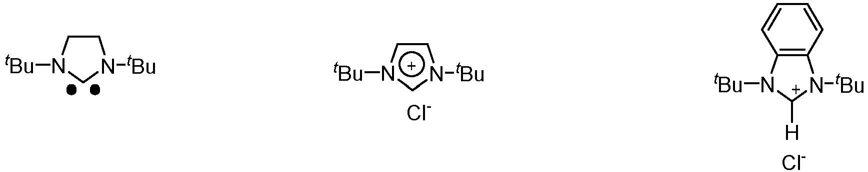 Illustrative and non-limiting examples of metalalactone complexes (representative nickelalactones) suitable for use as described herein include the following compounds (Cy = cyclohexyl, ^{t}Bu = tert-butyl):

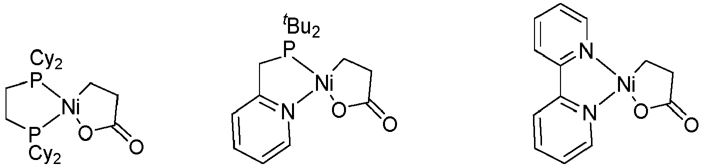

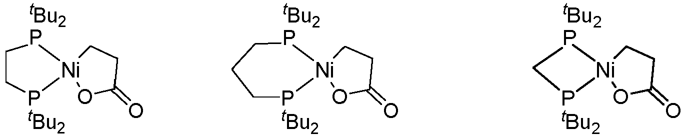

The transition metal precursor compounds corresponding to these illustrative metalalactones are shown below:

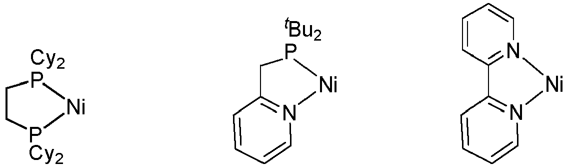

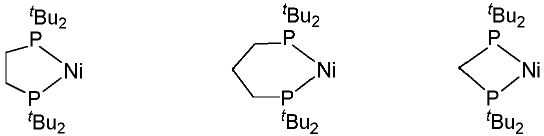

Metalalactones can be synthesized according to the following general reaction scheme (illustrated with nickel as the transition metal; Ni(COD)₂ is bis(1,5-cyclooctadiene)nickel(0)), and according to suitable procedures well known to those of skill in the art.

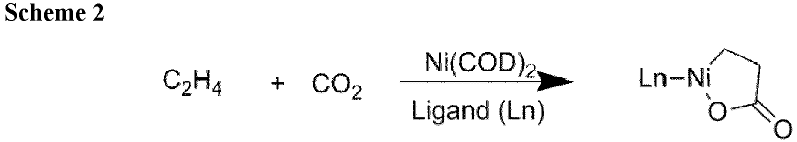

Suitable ligands, transition metal precursor compounds, and metalalactones are not limited solely to those ligands, transition metal precursor compounds, and metalalactones disclosed herein. Other suitable ligands, transition metal precursor compounds, and metalalactones are described, for example, in U.S. Patent Nos. 7,250,510, 8,642,803, and 8,697,909; Journal of Organometallic Chemistry, 1983, 251, C51-C53; Z. Anorg. Allg. Chem., 1989, 577, 111-114; Journal of Organometallic Chemistry, 2004, 689, 2952-2962; Organometallics, 2004, Vol. 23, 5252-5259; Chem. Commun., 2006, 2510-2512; Organometallics, 2010, Vol. 29, 2199-2202; Chem. Eur. J., 2012, 18, 14017-14025; Organometallics, 2013, 32 (7), 2152-2159; and Chem. Eur. J., 2014, Vol. 20, 11, 3205-3211.

The following references provide information related to the structure and/or activity relationships in the olefin and CO₂ coupling process, as observed by changes in phenoxide structure, the phosphine ligand structure, and other ligand structures: Manzini, S.; Huguet, N.; Trapp, O.; Schaub, T. Eur. J. Org. Chem. 2015, 7122; and Al-Ghamdi, M.; Vummaleti, S. V. C.; Falivene, L.; Pasha, F. A.; Beetstra, D. J.; Cavallo, L. Organometallics 2017, 36, 1107-1112.

By adjusting the basic particulate template size, molar ratio of formaldehyde to phenol monomer, polymerization conditions, the properties of the porous crosslinked polyphenoxide resin co-catalyst can be adjusted which can, in turn, provide higher turnover numbers. For example, the sodium site density and/or polymer surface area can be increased or maximized to provide higher turnovers. The pore size and/or pore density can be increased or adjusted to accommodate larger metalalactone intermediates that provide specific acrylates including various substituted acrylates.

Generally, the features of the processes disclosed herein (e.g., the metalalactone, the diluent, the porous crosslinked polyphenol and polyphenoxide resin, the α,β-unsaturated carboxylic acid or salt thereof, the transition metal precursor compound, the olefin, and the reaction conditions under which the α,β-unsaturatedcarboxylic acid, or a salt thereof, is formed, among others) are independently described, and these features can be combined in any combination to further describe the disclosed processes.

The present invention also provides a process for performing a metalalactone elimination reaction is disclosed, in which the process forms an α,β-unsaturated carboxylic acid or salt thereof. This process comprises (or consist essentially of, or consist of):
a) contacting
   1) a metalalactone compound;
   2) a diluent; and
   3) a promoter comprising a porous crosslinked polyphenoxide resin comprising associated metal cations to provide a reaction mixture; and
b) applying reaction conditions to the reaction mixture suitable to induce a metalalactone elimination reaction to form the α,β-unsaturated carboxylic acid or the salt thereof.

Suitable metalalactones, diluents, and porous crosslinked polyphenol resins are disclosed hereinabove. In this process for performing a metalalactone elimination reaction, for instance, at least a portion of the diluent can comprise the α,β-unsaturated carboxylic acid, or the salt thereof, that is formed in step (2) of this process.

In accordance with another aspect of the present disclosure, a process for producing an α,β-unsaturated carboxylic acid, or a salt thereof, is disclosed. This process can comprise (or consist essentially of, or consist of):
(1) contacting
   (a) a metalalactone compound;
   (b) a diluent; and
   (c) a porous crosslinked polyphenoxide resin comprising associated metal cations to provide a reaction mixture comprising an adduct of the metalalactone compound and the porous crosslinked polyphenoxide resin and its associated metal cations; and
(2) applying reaction conditions or process conditions to the reaction mixture suitable to form the α,β-unsaturated carboxylic acid or a salt thereof.
In this process for producing an α,β-unsaturated carboxylic acid or a salt thereof, for instance, at least a portion of the diluent of the reaction mixture comprising the adduct of the metalalactone can be removed after step (1), and before step (2), of this process. Suitable metalalactones, diluents, and porous crosslinked polyphenol resins are disclosed hereinabove.

As discussed further in this disclosure, the above processes can further comprise a step of contacting a transition metal precursor compound comprising at least one first ligand, an olefin, and carbon dioxide (CO₂) to form the metalalactone compound. That is, at least one ligand of the transition metal precursor compound can be carried over to the metalalactone compound. In further aspects, the above processes can further comprise a step of contacting a transition metal precursor compound comprising at least one first ligand with at least one second ligand, an olefin, and carbon dioxide (CO₂) to form the metalalactone compound. In this aspect, the ligand set of the metalalactone typically comprises the at least one ligand in addition to the metalalactone moiety. That is, the metalalactone compound can comprise the at least one first ligand, the at least one second ligand, or a combination thereof.

In some aspects, the contacting step - step (1) - of the above processes can include contacting, in any order, the metalalactone, the diluent, and the porous crosslinked polyphenoxide resin, and additional unrecited materials. In other aspects, the contacting step can consist essentially of, or consist of, the metalalactone, the diluent, and the porous crosslinked polyphenoxide resin components. Likewise, additional materials or features can be employed in the applying reaction conditions step - step (2) - that forms or produces the α,β-unsaturated carboxylic acid, or the salt thereof. Further, it is contemplated that these processes for producing an α,β-unsaturated carboxylic acid or a salt thereof by a metalalactone elimination reaction can employ more than one metalalactone and/or more than one porous crosslinked polyphenoxide resin. Additionally, a mixture or combination of two or more diluents can be employed.

Any suitable reactor, vessel, or container can be used to contact the metalalactone, diluent, and porous crosslinked polyphenoxide resin, non-limiting examples of which can include a flow reactor, a continuous reactor, a fixed bed reactor, a moving reactor bed, and a stirred tank reactor, including more than one reactor in series or in parallel, and including any combination of reactor types and arrangements. In particular aspects consistent with this disclosure, the metalalactone and the diluent can contact a fixed bed of the porous crosslinked polyphenoxide resin, for instance, in a suitable vessel, such as in a continuous fixed bed reactor. In further aspects, combinations of more than one porous crosslinked polyphenoxide resin can be used, such as a mixed bed of a first porous crosslinked polyphenoxide resin and a second porous crosslinked polyphenoxide resin, or sequential beds of a first porous crosslinked polyphenoxide resin and a second porous crosslinked polyphenoxide resin. In these and other aspects, the feed stream can flow upward or downward through the fixed bed. For instance, the metalalactone and the diluent can contact the first porous crosslinked polyphenoxide resin and then the second porous crosslinked polyphenoxide resin in a downward flow orientation, and the reverse in an upward flow orientation. In a different aspect, the metalalactone and the porous crosslinked polyphenoxide resin can be contacted by mixing or stirring in the diluent, for instance, in a suitable vessel, such as a stirred tank reactor.

Step (1) of the process for producing an α,β-unsaturated carboxylic acid or a salt thereof also recites forming an adduct of the metalalactone and the porous crosslinked polyphenoxide resin and its associated metal cations. Without intending to be bound by theory, there is some interaction between the metalalactone and the porous crosslinked polyphenoxide resin and its associated metal cations that are believed to destabilize the metalalactone for its elimination of the metal acrylate. This interaction can be referred to generally as an adduct of the metalalactone and the porous crosslinked polyphenoxide resin or an adduct of the α,β-unsaturated carboxylic acid with the porous crosslinked polyphenoxide resin. This adduct can contain all or a portion of the α,β-unsaturated carboxylic acid and can be inclusive of salts of the α,β-unsaturated carboxylic acid.

Accordingly, applying reaction conditions or process conditions to the reaction mixture suitable to form an α,β-unsaturated carboxylic acid or a salt thereof is intended to reflect any concomitant or subsequent reaction conditions to step (1) of the above processes that release the α,β-unsaturated carboxylic acid or a salt thereof from the adduct, regardless of the specific nature of the adduct.

For example, in step (2) of the process of applying reaction conditions or process conditions to the reaction mixture suitable to form an α,β-unsaturated carboxylic acid or a salt thereof, the adduct of the metalalactone and the porous crosslinked polyphenoxide resin and its associated metal cations as defined herein are subjected to some chemical or other reaction conditions or treatment to produce the α,β-unsaturated carboxylic acid or its salt. Various methods can be used to liberate the α,β-unsaturated carboxylic acid or its salt, from the porous crosslinked polyphenoxide resin. In one aspect, for instance, the treating step can comprise contacting the adduct of the metalalactone and the porous crosslinked polyphenoxide resin and its associated metal cations with an acid. Representative and non-limiting examples of suitable acids can include HCl, acetic acid, as well as combinations thereof. In another aspect, the treating step can comprise contacting the adduct of the metalalactone and the porous crosslinked polyphenoxide resin and its associated metal cations with a base. Representative and non-limiting examples of suitable bases can include carbonates (e.g., Na₂CO₃, Cs₂CO₃, MgCO₃), hydroxides (e.g., Mg(OH)₂, Na(OH), alkoxides (e.g., Al(O*ⁱ*Pr)₃, Na(O*^{t}*Bu), Mg(OEt)₂), as well as combinations thereof (*ⁱ*Pr = isopropyl, *^{t}*Bu = tert-butyl, Et = ethyl). In yet another aspect, the treating step can comprise contacting the adduct of the metalalactone and the porous crosslinked polyphenoxide resin and its associated metal cations with a suitable solvent. Representative and non-limiting examples of suitable solvents can include carbonyl-containing solvents such as ketones, esters, amides, etc. (e.g., acetone, ethyl acetate, N,N-dimethylformamide, etc., as described herein above), alcohol solvents, water, as well as combinations thereof.

In still another aspect, the treating step can comprise heating the adduct of the metalalactone and the porous crosslinked polyphenoxide resin and its associated metal cations to any suitable temperature. This temperature can be in a range, for example, from 50 to 1000 °C, from 100 to 800 °C, from 150 to 600 °C, from 250 to 1000 °C, from 250 °C to 550 °C, or from 150 °C to 500 °C. The duration of this heating step is not limited to any particular period of time, as long of the period of time is sufficient to liberate the α,β-unsaturated carboxylic acid from the porous crosslinked polyphenoxide resin. As those of skill in the art recognize, the appropriate treating step depends upon several factors, such as the particular diluent used in the process, and the particular porous crosslinked polyphenoxide resin used in the process, amongst other considerations. One further treatment step can comprise, for example, a workup step with additional olefin to displace an alkene-nickel bound acrylate.

In these processes for performing a metalalactone elimination reaction and for producing an α,β-unsaturated carboxylic acid (or a salt thereof), additional process steps can be conducted before, during, and/or after any of the steps described herein. As an example, these processes can further comprise a step (e.g., prior to step (1)) of contacting a transition metal precursor compound with an olefin and carbon dioxide to form the metalalactone. Transition metal precursor compound are described hereinabove. Illustrative and non-limiting examples of suitable olefins can include ethylene, propylene, butene (e.g., 1-butene), pentene, hexene (e.g., 1-hexene), heptane, octene (e.g., 1-octene), and styrene, as well as combinations thereof.

The present invention provides a process for producing an α,β-unsaturated carboxylic acid, or a salt thereof, is disclosed. This process comprises (or consist essentially of, or consist of):
(1) contacting in any order
   (a) a transition metal precursor compound comprising at least one first ligand;
   (b) optionally, at least one second ligand;
   (c) an olefin;
   (d) carbon dioxide (CO₂);
   (e) a diluent; and
   (f) a porous crosslinked polyphenoxide resin comprising associated metal cations to provide a reaction mixture; and
(2) applying reaction conditions to the reaction mixture suitable to form an α,β-unsaturated carboxylic acid or a salt thereof.

In aspects of this process that utilizes a transition metal precursor compound comprising at least one first ligand, the olefin can be ethylene, and the step of contacting a transition metal precursor compound with an olefin and carbon dioxide (CO₂) can be conducted using any suitable pressure of ethylene, or any pressure of ethylene disclosed herein, e.g., from 10 psig (70 KPa) to 1,000 psig (6,895 KPa), from 25 psig (172 KPa) to 500 psig (3,447 KPa), or from 50 psig (345 KPa) to 300 psig (2,068 KPa). Further, the olefin can be ethylene, and the step of contacting a transition metal precursor compound with an olefin and carbon dioxide (CO₂) can be conducted using a constant addition of the olefin, a constant addition of carbon dioxide, or a constant addition of both the olefin and carbon dioxide, to provide the reaction mixture. By way of example, in a process wherein the ethylene and carbon dioxide (CO₂) are constantly added, the process can utilize an ethylene:CO₂ molar ratio of from 5:1 to 1:5, from 3:1 to 1:3, from 2:1 to 1:2, or about 1:1, to provide the reaction mixture.

According to a further aspect of the above process that utilizes a transition metal precursor compound, the process can include the step of contacting a transition metal precursor compound with an olefin and carbon dioxide (CO₂) conducted using any suitable pressure of CO₂, or any pressure of CO₂ disclosed herein, e.g., from 20 psig (138 KPa) to 2,000 psig (13,790 KPa), from 50 psig (345 KPa) to 750 psig (5,171 KPa), or from 100 psig (689 KPa) to 300 psig (2,068 KPa). In any of the processes disclosed herein, the processes can further comprise a step of monitoring the concentration of at least one reaction mixture component, at least one elimination reaction product, or a combination thereof, for any reason, such as to adjust process parameters in real time, to determine extent or reaction, or to stop the reaction at the desired point.

As illustrated, this process that utilizes a transition metal precursor compound comprising at least one first ligand includes one aspect in which no second ligand is employed in the contacting step, and another aspect in which a second ligand is used in the contacting step. That is, one aspect involves the contacting step of the process comprising contacting the transition metal precursor compound comprising at least one first ligand with the at least one second ligand. The order of contacting can be varied. For example, the contacting step of the process disclosed above can comprise contacting (a) the transition metal precursor compound comprising at least one first ligand with (b) the at least one second ligand to form a pre-contacted mixture, followed by contacting the pre-contacted mixture with the remaining components (c)-(f) in any order to provide the reaction mixture.

Further aspects or embodiments related to the order of contacting, for example, the contacting step can include or comprise contacting the metalalactone, the diluent, and the porous crosslinked polyphenoxide resin in any order. The contacting step can also comprise contacting the metalalactone and the diluent to form a first mixture, followed by contacting the first mixture with the porous crosslinked polyphenoxide resin to form the reaction mixture. In a further aspect, the contacting step can comprise contacting the diluent and the porous crosslinked polyphenoxide resin to form a first mixture, followed by contacting the first mixture with the metalalactone to form the reaction mixture. In yet a further aspect, the contacting step of the process can further comprise contacting any number of additives, for example, additives that can be selected from an acid, a base, or a reductant.

Suitable transition metal-ligand complexes, olefins, diluents, porous crosslinked polyphenoxide resins comprising associated metal cations are disclosed hereinabove. In some aspects, the contacting step - step (1) - of this process can include contacting, in any order, the transition metal-ligand complexes, the olefin, the diluent, the porous crosslinked polyphenoxide resin and carbon dioxide, and additional unrecited materials. In other aspects, the contacting step can consist essentially of, or consist of, contacting, in any order, the transition metal-ligand complex, the olefin, the diluent, the porous crosslinked polyphenoxide resin, and carbon dioxide. Likewise, additional materials or features can be employed in the forming step of step (2) of this process. Further, it is contemplated that this processes for producing an α,β-unsaturated carboxylic acid, or a salt thereof, can employ more than one transition metal-ligand complex and/or more than one porous crosslinked polyphenoxide resin if desired and/or more than one olefin. Additionally, a mixture or combination of two or more diluents can be employed.

As above, any suitable reactor, vessel, or container can be used to contact the transition metal-ligand complex, olefin, diluent, porous crosslinked polyphenoxide resin, and carbon dioxide, whether using a fixed bed of the porous crosslinked polyphenoxide resin, a stirred tank for contacting (or mixing), or some other reactor configuration and process. While not wishing to be bound by the following theory, a proposed and illustrative reaction scheme for this process is provided below. 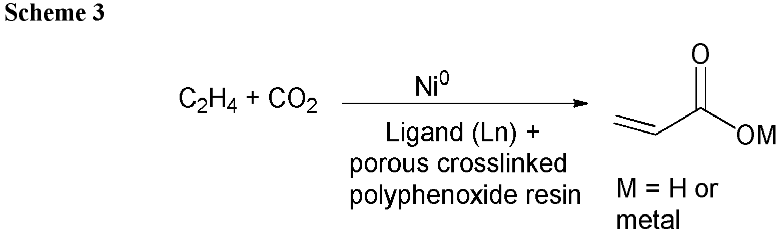 Independently, the contacting and forming steps of any of the processes disclosed herein (i.e., for performing a metalalactone elimination reaction, for producing an α,β-unsaturated carboxylic acid, or a salt thereof), can be conducted at a variety of temperatures, pressures, and time periods. For instance, the temperature at which the components in step (1) are initially contacted can be the same as, or different from, the temperature at which the forming step (2) is performed. As an illustrative example, in the contacting step, the components can be contacted initially at temperature T1 and, after this initial combining, the temperature can be increased to a temperature T2 for the forming step (e.g., to form the α,β-unsaturated carboxylic acid, or the salt thereof). Likewise, the pressure can be different in the contacting step and the forming step. Often, the time period in the contacting step can be referred to as the contact time, while the time period in forming step can be referred to as the reaction time. The contact time and the reaction time can be, and often are, different.

In an aspect, the contacting step and/or the forming step of the processes disclosed herein can be conducted at a temperature in a range from 0 °C to 250 °C; alternatively, from 20 °C to 200 °C; alternatively, from 0 °C to 95 °C; alternatively, from 10 °C to 75 °C; alternatively, from 10 °C to 50 °C; or alternatively, from 15 °C to 70 °C. In these and other aspects, after the initial contacting, the temperature can be changed, if desired, to another temperature for the forming step. These temperature ranges also are meant to encompass circumstances where the contacting step and/or the forming step can be conducted at a series of different temperatures, instead of at a single fixed temperature, falling within the respective ranges.

In an aspect, the contacting step and/or the forming step of the processes disclosed herein can be conducted at a pressure in a range from 5 (34 KPa) to 10,000 psig (68,948 KPa), such as, for example, from 5 psig (34 KPa) to 2500 psig (17,237 KPa). In some aspects, the pressure can be in a range from 5 psig (34 KPa) to 500 psig (3,447 KPa); alternatively, from 25 psig (172 KPa) to 3000 psig (20,684 KPa); alternatively, from 45 psig (310 KPa) to 1000 psig (6,895 KPa); or alternatively, from 50 psig (345 KPa) to 250 psig (1,724 KPa).

The contacting step of the processes is not limited to any particular duration of time. That is, the respective components can be initially contacted rapidly, or over a longer period of time, before commencing the forming step. Hence, the contacting step can be conducted, for example, in a time period ranging from as little as 1-30 seconds to as long as 1-12 hours, or more. In non-continuous or batch operations, the appropriate reaction time for the forming step can depend upon, for example, the reaction temperature, the reaction pressure, and the ratios of the respective components in the contacting step, among other variables. Generally, however, the forming step can occur over a time period that can be in a range from 1 minute to 96 hours, such as, for example, from 2 minutes to 96 hours, from 5 minutes to 72 hours, from 10 minutes to 72 hours, or from 15 minutes to 48 hours.

If the process employed is a continuous process, then the metalalactone/anionic electrolyte catalyst contact/reaction time (or the transition metal-ligand complex/anionic electrolyte catalyst contact/reaction time) can be expressed in terms of weight hourly space velocity (WHSV)-the ratio of the weight per unit time (for example, g/hr) of the metalalactone (or transition metal-ligand complex) containing solution which comes in contact with a given weight (for example, g) of anionic electrolyte per unit time. While not limited thereto, the WHSV employed, based on the amount of the anionic electrolyte, can be in a range from 0.05 to 100 hr⁻¹, from 0.05 to 50 hr⁻¹, from 0.075 to 50 hr⁻¹, from 0.1 to 25 hr⁻¹, from 0.5 to 10 hr⁻¹, from 1 to 25 hr⁻¹, or from 1 to 5 hr⁻¹.

In the processes disclosed herein, the molar yield of the α,β-unsaturated carboxylic acid, or the salt thereof), based on the metalalactone (or the transition metal-ligand complex) is at least 2%, and more often can be at least 5%, at least 10%, or at least 15%. In particular aspects of this disclosure, the molar yield can be at least 18%, at least 20%, at least 25%, at least 35%, at least 50%, at least 60%, at least 75%, or at least 85%, or at least 90%, or at least 95%, or at least 100%. That is, catalytic formation of the α,β-unsaturated carboxylic acid or the salt thereof can be effected with the disclosed system. For example, the molar yield of the α,β-unsaturated carboxylic acid, or the salt thereof, based on the metalalactone or based on the transition metal precursor compound can be at least 20%, at least 40%, at least 60%, at least 80%, at least 100%, at least 120%, at least 140%, at least 160%, at least 180%, at least 200%, at least 250%, at least 300%, at least 350%, at least 400%, at least 450%, or at least 500%.

The specific α,β-unsaturated carboxylic acid (or salt thereof) that can be formed or produced using the processes of this disclosure is not particularly limited. Illustrative and non-limiting examples of the α,β-unsaturated carboxylic acid can include acrylic acid, methacrylic acid, 2-ethylacrylic acid, cinnamic acid, as well as combinations thereof. Illustrative and non-limiting examples of the salt of the α,β-unsaturated carboxylic acid can include sodium acrylate, potassium acrylate, magnesium acrylate, sodium (meth)acrylate, as well as combinations thereof.

Once formed, the α,β-unsaturated carboxylic acid (or salt thereof) can be purified and/or isolated and/or separated using suitable techniques which can include evaporation, distillation, chromatography, crystallization, extraction, washing, decanting, filtering, drying, including combinations of more than one of these techniques. In an aspect, the process can for performing a metalalactone elimination reaction (or the process for producing an α,β-unsaturated carboxylic acid, or a salt thereof) can further comprise a step of separating or isolating the α,β-unsaturated carboxylic acid (or salt thereof) from other components, e.g., the diluent, the anionic electrolyte.

### EXAMPLES

The invention is further illustrated by the following examples, which are not to be construed in any way as imposing limitations to the scope of this invention. Various other aspects, embodiments, modifications, and equivalents thereof which, after reading the description herein, can suggest themselves to one of ordinary skill in the art without departing from the scope of the appended claims.

### General Considerations

Unless otherwise noted, all operations were performed under purified nitrogen or vacuum using standard Schlenk or glovebox techniques. Toluene (Honeywell) and tetrahydrofuran (Aldrich) was degassed and dried over activated 4Å molecular sieves under nitrogen. Sodium tert-butoxide and potassium tert-butoxide were purchased from Sigma-Aldrich and used as received. Phenol-formaldehyde resin was purchased as hollow beads ( ~5-127 µm) from Polysciences, Inc. Bis(1,5-cyclooctadiene)nickel(0) and 1,2-Bis-(dicyclohexylphosphino)ethane were purchased from Strem and were used as received. (TMEDA)Ni(CH₂CH₂CO₂) was prepared according to literature procedures (Fischer, R; Nestler, B., and Schutz, H. Z. anorg. allg. Chem. 577 (1989) 111-114).

### Preparation of Compounds

**Sodium phenol-formaldehyde resin.** Phenolic resin (phenol-formaldehyde resin) was suspended in a solution of sodium hydroxide in either water or methanol and stirred at 55 °C overnight prior to filtration, and subsequently washed with copious amounts of the solvent in which it was treated. The solid was then dried under vacuum prior to storage under nitrogen.

### EXAMPLE 1-3

### Sodium-Treated Crosslinked Polyphenoxide Resins as Stoichiometric Co-catalysts in Olefin/Carbon Dioxide Conversion to α,β-Unsaturated Carboxylates

These examples describe the formation of a crosslinked polyphenoxide resin that is prepared in a non-templated fashion, for comparison with the templated resins. It was believed that these crosslinked polyaromatic resins would be sufficiently insoluble in many commercial diluents to be applicability as a polymeric promoters and cation sources in a fixed bed/column reactor setting. This method further allows for the potential regeneration of the spent solid co-catalyst in both aqueous (for example, sodium hydroxide in water) and/or organic media (for example, sodium alkoxide in toluene).

The following Scheme illustrates the conversion reaction of an olefin and carbon dioxide-derived nickelalactone intermediate that was undertaken to evaluate some crosslinked polyelectrolyte analogues. Reaction conditions for reaction (3) are: 0.10 mmol [Ni], 0.11 mmol diphosphine ligand, 500 mL of toluene, 1.0 g of sodium-treated, crosslinked polyaromatic resin (solid activator). The reactor was equilibrated to 150 psi of ethylene followed by 300 psi of carbon dioxide prior to heating. The yield reported in Table 3 was determined by ¹H NMR spectroscopy in a D₂O/(CD₃)₂CO mixture relative to a sorbic acid standard.

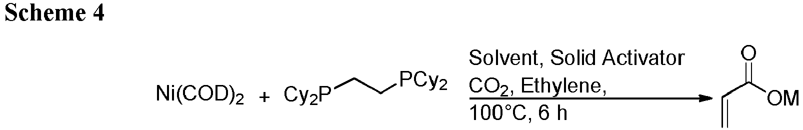

The following table describe various examples where commercial polyaromatic resins, which were either further treated with a sodium base under appropriate conditions or are commercially available in the sodium form, were found to be effective in the nickel-mediated synthesis of sodium acrylate from ethylene and carbon dioxide.

**Table 3. Nickel-mediated conversion of carbon dioxide and ethylene to sodium acrylate with sodium treated polyaromatics.^{A}**

| **Example** | **Solvent** | **Co-catalyst Solid** | **Base & Sodium Source** | **[Solid]:[Na] (wt)** | **Acrylate yield (%)** |
|---|---|---|---|---|---|
| 1 | toluene | Phenol-Formaldehyde | NaOH (MeOH) | 0.3 | 1.8 |
| 2 | toluene | Phenol-Formaldehyde | NaOH (aq) | 0.3 | 6.0 |
| 3 | toluene | Phenol-Formaldehyde | NaO-t-Bu | 1.0 | n.d.^{B} |

| | | | | | |
|---|---|---|---|---|---|
| ^{A} Conditions: 0.10 mmol [Ni], 0.11 mmol diphosphine ligand, 500 mL toluene, 1.0 g solid activator (phenol-formaldehyde resin). Reactor was equilibrated to 150 psi ethylene followed by 300 psi carbon dioxide prior to heating. Yield determined by ¹H NMR spectroscopy in D₂O/(CD₃)₂CO mixture relative to sorbic acid standard. ^{B} None detected. | | | | | |

Among other things, Examples 1-3 illustrate the effect that porosity has on base treatment and ultimately on the acrylate yield. For example, the highest acrylate yield was observed with hydroxide base, whereas the lowest (none detected) yield was observed with the bulky t-butoxide base.

### EXAMPLE 4

### Templated and Non-Templated Crosslinked Polyphenoxide Resin Co-catalysts in Olefin/Carbon Dioxide Conversion to α,β-Unsaturated Carboxylates, using Co-monomers

In this example, co-monomer phenol compounds are used together with formaldehyde to prepare the crosslinked polyaromatic resins for use as described according to the disclosure. This reaction can also be carried out in a templated fashion in the presence of CaCO₃ as the basic particulate template, for example, to form the more porous form of the crosslinked polyphoxide resin described here.

This non-templated resin was prepared using the co-monomer combination of resorcinol (m-dihydroxybenzene) and 2-fluorophenol monomer with formaldehyde, and the resulting resin was sodium-treated (NaOH, dissolved in water or alcohol) to generate the porous crosslinked polyphenol resin, according to the following reaction scheme.

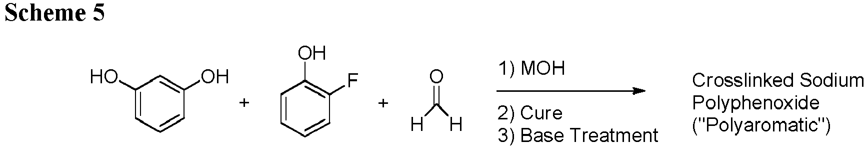

The polyaromatic resin is thought to act as a co-catalyst upon treatment with sodium hydroxide because of what are believed to be sodium aryloxide sites that promote nickelalactone scission. It is noted that increased crosslink density is obtained using longer drying times to remove trapped excess water.

This process can be carried out using the templating process described above in the presence of CaCO₃ or MgCO₃ to provide a highly porous resin.

### EXAMPLE 5-6

### Porous Crosslinked Polyphenoxide Resins as Co-catalysts in Olefin/Carbon Dioxide Conversion to α,β-Unsaturated Carboxylates

These examples describe the formation of a crosslinked polyphenoxide resins of Examples 1-2 using the templating technique described herein. This process not only provided insoluble resins that allowed ease of separation of the α,β-unsaturated carboxylate from the co-catalyst, but also provided the porous crosslinked structure that allowed for high sodium deposition density and facile sodium site access by the metalalactone.

A porous crosslinked polyphenoxide resin was formed by the following process. A CaCO₃ basic particulate template is provided, and contacted with at least one phenol compound, formaldehyde, and an aqueous base (NaOH(aq)) under polymerization conditions sufficient to form a templated crosslinked polyphenol resin, in which the crosslinked polyphenol resin formed in the reaction is in contact with the basic particulate template. This templated crosslinked polyphenol resin in contact with the CaCO₃ is then contacted with HCl(aq), which removes the basic particulate template and forms the porous crosslinked polyphenol resin in the absence of the template used in its formation. Finally, this porous crosslinked polyphenol resin is then contacted with a metal-containing base such as NaOH in methanol of NaOH(aq) to form a co-catalyst or promoter comprising a porous crosslinked polyphenoxide resin with associated sodium cations.

The porous crosslinked polyphenoxide co-catalyst is used as the solid activator in reaction (3) illustrated above, to convert an olefin and carbon dioxide-derived nickelalactone intermediate to the sodium acrylate, according to the procedure set out in Examples 1-3 and illustrated in the following table.

**Table 3. Nickel-mediated conversion of carbon dioxide and ethylene to sodium acrylate with sodium treated polyaromatics.^{A}**

| **Example** | **Solvent** | **Templated Co-catalyst Solid** | **Base & Sodium Source** | **[Solid]:[Na] (wt)** |
|---|---|---|---|---|
| 5 | toluene | Phenol-Formaldehyde | NaOH (MeOH) | 0.3 |
| 6 | toluene | Phenol-Formaldehyde | NaOH (aq) | 0.3 |

| | | | | |
|---|---|---|---|---|
| ^{A} Conditions: 0.10 mmol [Ni], 0.11 mmol diphosphine ligand, 500 mL toluene, 1.0 g solid activator (phenol-formaldehyde resin). Reactor was equilibrated to 150 psi ethylene followed by 300 psi carbon dioxide prior to heating. Yield determined by ¹H NMR spectroscopy in D₂O/(CD₃)₂CO mixture relative to sorbic acid standard. | | | | |

Even though the yields of acrylate when employing these sodium-treated crosslinked resins may be modest, the examples indicate that the nickel-mediated conversion of carbon dioxide and ethylene to sodium acrylate with sodium treated crosslinked polyaromatic resins can be carried out. Further, the insolubilities of these resins in many commercial solvents will allow for their utility in fixed bed/column configurations.

The invention is described above with reference to numerous aspects and embodiments, and specific examples. Many variations will suggest themselves to those skilled in the art in light of the above detailed description. All such obvious variations are within the full intended scope of the appended claims.

## Claims

1. A process for forming an α,β-unsaturated carboxylic acid or a salt thereof, the process comprising:
a) contacting in any order
1) a transition metal precursor compound comprising at least one first ligand;
2) optionally, at least one second ligand;
3) an olefin;
4) carbon dioxide (CO₂);
5) a diluent; and
6) a promoter comprising a porous crosslinked polyphenoxide resin comprising associated metal cations, to provide a reaction mixture; and
b) applying reaction conditions to the reaction mixture suitable to form the α,β-unsaturated carboxylic acid or the salt thereof.

2. A process for forming an α,β-unsaturated carboxylic acid or a salt thereof, the process comprising:
a) contacting
1) a metalalactone compound;
2) a diluent; and
3) a promoter comprising a porous crosslinked polyphenoxide resin comprising associated metal cations to provide a reaction mixture; and
b) applying reaction conditions to the reaction mixture suitable to induce a metalalactone elimination reaction to form the α,β-unsaturated carboxylic acid or the salt thereof.

3. A process for forming a porous crosslinked polyphenoxide resin, the process comprising:
a) in the presence of a basic particulate template, contacting at least one phenol compound, formaldehyde, and an aqueous base under polymerization conditions sufficient to form a templated crosslinked polyphenol resin comprising a crosslinked polyphenol resin in contact with the basic particulate template;
b) contacting the templated crosslinked polyphenol resin with an aqueous acid under pore forming conditions sufficient to remove the basic particulate template and form a porous crosslinked polyphenol resin; and
c) contacting the porous crosslinked polyphenol resin with a metal-containing base to form a promoter comprising a porous crosslinked polyphenoxide resin comprising associated metal cations.

4. The process according to claims 1, 2 or 3, wherein the porous crosslinked polyphenoxide resin comprises a phenoxide-formaldehyde resin, a polyhydroxidearene-formaldehyde resin, a polyhydroxidearene- and fluorophenoxide-formaldehyde resin, or combinations thereof.

5. The process according to claims 1 or 2, wherein the associated metal cations are selected from a Group 1, 2, 12, or 13 metal.

6. The process according to claims 1 or 2, wherein the porous crosslinked polyphenoxide resin has an average pore diameter of from about 2 nm to about 250 nm.

7. The process according to claims 1 or 2, wherein the diluent comprises an aromatic hydrocarbon solvent, an ether solvent, a carbonyl-containing solvent, a halogenated aromatic hydrocarbon solvent, carbon dioxide, or an α,β-unsaturatedcarboxylic acid or the salt thereof.

8. The process according to claims 1 or 2, wherein the reaction conditions comprise contacting the reaction mixture with a metal-containing base, optionally wherein the metal-containing base is selected from an alkali metal or an alkaline earth metal oxide, hydroxide, alkoxide, aryloxide, amide, alkyl amide, arylamide, or carbonate.

9. The process according to claim 1, wherein the olefin is ethylene, and wherein
the step of contacting the transition metal precursor with the olefin and carbon dioxide (CO₂) is conducted using from 10 psig (689 KPa) to 1,000 psig (6,902 KPa) of ethylene partial pressure and/or from 20 psig (138 KPa) to 2,000 psig (13,790 KPa) of CO₂ partial pressure; or
the ethylene and carbon dioxide are added in a constant or a variable ethylene:CO₂ molar ratio of from 10:1 to 1:10, to provide the reaction mixture.

10. The process according to claims 1 or 2, wherein the transition metal precursor compound comprises a group 8-11 transition metal.

11. The process according to claims 1 or 2, wherein the porous crosslinked polyphenoxide resin of the contacting step a) comprises a fixed bed.

12. The process according to claims 1 or 2, wherein the contacting step and/or the applying step is conducted at a weight hourly space velocity (WHSV) of from 0.05 to 50 hr⁻¹, based on the amount of the porous crosslinked polyphenoxide resin, and at temperature of from 0 °C to 250 °C.

13. The process according to claims 1 or 2, the process further comprising a step of isolating the α,β-unsaturated carboxylic acid, or the salt thereof.

14. The process according to claim 1, further comprising the step of regenerating the porous crosslinked polyphenoxide resin by contacting a porous crosslinked polyphenol resin that is generated from the process with a base comprising a metal cation, or by contacting a porous crosslinked polyphenol resin that is generated from the process with a metal-containing salt.

15. The process according to claim 3, wherein the basic particulate template has a solubility in water of less than about 0.25 g/L at 25 °C or an average particle size from about 2 µm (micrometers) to about 200 µm.

## Patentansprüche

1. Vorgang zum Herstellen einer a,β-ungesättigten Carbonsäure oder eines Salzes davon, umfassend:
a) Inberührungbringen in beliebiger Reihenfolge
1) einer Übergangsmetallvorläuferverbindung, die mindestens einen ersten Liganden umfasst;
2) optional mindestens eines zweiten Liganden;
3) eines Olefins;
4) Kohlendioxids (CO₂);
5) eines Verdünnungsmittels; und
6) eines Promotors, der ein poröses, vernetztes Polyphenoxidharz umfasst, das assoziierte Metallkationen umfasst, um ein Reaktionsgemisch bereitzustellen; und
b) Anwenden von Reaktionsbedingungen auf das Reaktionsgemisch, um die a,β-ungesättigte Carbonsäure oder das Salz davon herzustellen.

2. Vorgang zum Herstellen einer a,β-ungesättigten Carbonsäure oder eines Salzes davon, wobei der Vorgang Folgendes umfasst:
a) Inberührungbringen
1) einer Metallalaktonverbindung;
2) eines Verdünnungsmittels; und
3) eines Promotors, der ein poröses, vernetztes Polyphenoxidharz umfasst, das assoziierte Metallkationen umfasst, um ein Reaktionsgemisch bereitzustellen; und
b) Anwenden von Reaktionsbedingungen auf das Reaktionsgemisch, die dazu geeignet sind, eine Metallalaktoneliminierungsreaktion auszulösen, um die α,β-ungesättigte Carbonsäure oder das Salz davon herzustellen.

3. Vorgang zum Herstellen eines porösen, vernetzten Polyphenoxidharzes, wobei der Vorgang Folgendes umfasst:
a) in Gegenwart einer basischen Partikelmatrize Inberührungbringen mindestens einer Phenolverbindung, Formaldehyds und einer wässrigen Base unter Polymerisationsbedingungen, die ausreichen, um ein matriziertes vernetztes Polyphenolharz herzustellen, das ein vernetztes Polyphenolharz in Berührung mit der basischen Partikelmatrize umfasst;
b) Inberührungbringen des matrizierten vernetzten Polyphenolharzes mit einer wässrigen Säure unter porenherstellenden Bedingungen, die ausreichen, um die basische Partikelmatrize zu entfernen und ein poröses vernetztes Polyphenolharz herzustellen; und
c) Inberührungbringen des porösen vernetzten Polyphenolharzes mit einer metallhaltigen Base, um einen Promotor herzustellen, der ein poröses vernetztes Polyphenolharz umfasst, das assoziierte Metallkationen umfasst.

4. Vorgang nach Anspruch 1, 2 oder 3, wobei das poröse vernetzte Polyphenoxidharz ein Phenoxidformaldehydharz, ein Polyhydroxidarenformaldehydharz, ein Polyhydroxidaren- und Fluorphenoxidformaldehydharz oder Kombinationen davon umfasst.

5. Vorgang nach Anspruch 1 oder 2, wobei die assoziierten Metallkationen aus einem Metall der Gruppe 1, 2, 12 oder 13 ausgewählt sind.

6. Vorgang nach Anspruch 1 oder 2, wobei das poröse vernetzte Polyphenoxidharz einen durchschnittlichen Porendurchmesser von etwa 2 nm bis etwa 250 nm aufweist.

7. Vorgang nach Anspruch 1 oder 2, wobei das Verdünnungsmittel ein aromatisches Kohlenwasserstofflösungsmittel, ein Etherlösungsmittel, ein carbonylhaltiges Lösungsmittel, ein halogeniertes aromatisches Kohlenwasserstofflösungsmittel, Kohlendioxid oder eine a,β-ungesättigte Carbonsäure oder das Salz davon umfasst.

8. Vorgang nach Anspruch 1 oder 2, wobei die Reaktionsbedingungen das Inberührungbringen des Reaktionsgemisches mit einer metallhaltigen Base umfassen, wobei die metallhaltige Base aus einem Alkalimetall- oder einem Erdalkalimetalloxid, -hydroxid, -alkoxid, -aryloxid, -amid, - alkylamid, -arylamid oder -carbonat ausgewählt ist.

9. Vorgang nach Anspruch 1, wobei das Olefin Ethylen ist und wobei
der Schritt des Inberührungbringens des Übergangsmetallvorläufers mit dem Olefin und dem Kohlendioxid (CO₂) unter Verwendung eines Ethylenpartialdrucks von 10 psig (689 kPa) bis 1.000 psig (6.902 kPa) und/oder eines CO₂-Partialdrucks von 20 psig (138 kPa) bis 2.000 psig (13.790 kPa) ausgeführt wird; oder
das Ethylen und das Kohlendioxid in einem konstanten oder einem variablen Ethylen:CO₂-Molverhältnis von 10:1 bis 1:10 hinzugefügt werden, um das Reaktionsgemisch bereitzustellen.

10. Vorgang nach Anspruch 1 oder 2, wobei die Übergangsmetallvorläuferverbindung ein Übergangsmetall der Gruppen 8-11 umfasst.

11. Vorgang nach Anspruch 1 oder 2, wobei das poröse, vernetzte Polyphenoxidharz des Inberührungbringschritts a) ein Festbett umfasst.

12. Vorgang nach Anspruch 1 oder 2, wobei der Inberührungbringschritt und/oder der Anwendungsschritt bei einer gewichteten stündlichen Raumgeschwindigkeit (WHSV) von 0,05 bis 50 h⁻¹, basierend auf der Menge des porösen vernetzten Polyphenoxidharzes, und bei einer Temperatur von 0 °C bis 250 °C ausgeführt wird.

13. Vorgang nach Anspruch 1 oder 2, wobei der Vorgang ferner einen Schritt von Isolieren der α,β-ungesättigten Carbonsäure oder des Salzes davon umfasst.

14. Vorgang nach Anspruch 1, ferner umfassend den Schritt von Regenerieren des porösen vernetzten Polyphenolharzes durch Inberührungbringen eines porösen vernetzten Polyphenolharzes, das durch den Vorgang erzeugt wird, mit einer Base, die ein Metallkation umfasst, oder durch Inberührungbringen eines porösen vernetzten Polyphenolharzes, das durch den Vorgang erzeugt wird, mit einem metallhaltigen Salz.

15. Vorgang nach Anspruch 3, wobei die basische Partikelmatrize eine Wasserlöslichkeit von weniger als etwa 0,25 g/l bei 25 °C oder eine durchschnittliche Partikelgröße von etwa 2 µm (Mikrometer) bis etwa 200 µm aufweist.

## Revendications

1. Procédé de formation d'un acide carboxylique α,β-insaturé ou d'un sel de celui-ci, le procédé comprenant :
a) la mise en contact dans n'importe quel ordre
1) d'un composé précurseur de métal de transition comprenant au moins un premier ligand ;
2) éventuellement, au moins un second ligand ;
3) une oléfine ;
4) du dioxyde de carbone (CO₂) ;
5) un diluant ; et
6) un promoteur comprenant une résine polyphénoxyde réticulée poreuse comprenant des cations métalliques associés, pour fournir un mélange réactionnel ; et
b) l'application de conditions de réaction au mélange réactionnel appropriées pour former l'acide carboxylique α,β-insaturé ou le sel de celui-ci.

2. Procédé de formation d'un acide carboxylique α,β-insaturé ou d'un sel de celui-ci, le procédé comprenant :
a) la mise en contact
1) d'un composé métalalactone ;
2) d'un diluant ; et
3) d'un promoteur comprenant une résine polyphénoxyde réticulée poreuse comprenant des cations métalliques associés pour fournir un mélange réactionnel ; et
b) l'application de conditions de réaction au mélange réactionnel appropriées pour induire une réaction d'élimination de métalalactone pour former l'acide carboxylique α,β-insaturé ou le sel de celui-ci.

3. Procédé de formation d'une résine polyphénoxyde réticulée poreuse, le procédé comprenant :
a) en présence d'un modèle particulaire basique, la mise en contact d'au moins un composé phénolique, du formaldéhyde et d'une base aqueuse dans des conditions de polymérisation suffisantes pour former une résine polyphénol réticulée modélisée comprenant une résine polyphénol réticulée en contact avec le modèle particulaire basique ;
b) la mise en contact de la résine polyphénol réticulée modélisée avec un acide aqueux dans des conditions de formation de pores suffisantes pour éliminer le modèle particulaire basique et former une résine polyphénol réticulée poreuse ; et
c) la mise en contact de la résine polyphénol réticulée poreuse avec une base contenant du métal pour former un promoteur comprenant une résine polyphénoxyde réticulée poreuse comprenant des cations métalliques associés.

4. Procédé selon les revendications 1, 2 ou 3, dans lequel la résine polyphénoxyde réticulée poreuse comprend une résine phénoxyde-formaldéhyde, une résine polyhydroxydéarène-formaldéhyde, une résine polyhydroxydéarène- et fluorophénoxyde-formaldéhyde, ou des combinaisons de celles-ci.

5. Procédé selon les revendications 1 ou 2, dans lequel les cations métalliques associés sont choisis parmi un métal du groupe 1, 2, 12 ou 13.

6. Procédé selon les revendications 1 ou 2, dans lequel la résine polyphénoxyde réticulée poreuse a un diamètre moyen de pores allant d'environ 2 nm à environ 250 nm.

7. Procédé selon les revendications 1 ou 2, dans lequel le diluant comprend un solvant hydrocarbure aromatique, un solvant éther, un solvant contenant du carbonyle, un solvant hydrocarbure aromatique halogéné, du dioxyde de carbone ou un acide carboxylique α,β-insaturé ou le sel de celui-ci.

8. Procédé selon les revendications 1 ou 2, dans lequel les conditions de réaction comprennent la mise en contact du mélange réactionnel avec une base contenant du métal, éventuellement dans lequel la base contenant du métal est choisie parmi un métal alcalin ou un oxyde, hydroxyde, alcoxyde, aryloxyde, amide, alkylamide, arylamide ou carbonate de métal alcalino-terreux.

9. Procédé selon la revendication 1, dans lequel l'oléfine est l'éthylène, et dans lequel
l'étape de mise en contact du précurseur de métal de transition avec l'oléfine et le dioxyde de carbone (CO₂) est réalisée à l'aide de 10 psig (689 KPa) à 1 000 psig (6 902 KPa) de pression partielle d'éthylène et/ou de 20 psig (138 KPa) à 2 000 psig (13 790 KPa) de pression partielle de CO₂ ; ou
l'éthylène et le dioxyde de carbone sont ajoutés selon un rapport molaire éthylène:CO₂ constant ou variable de 10:1 à 1:10, pour obtenir le mélange réactionnel.

10. Procédé selon les revendications 1 ou 2, dans lequel le composé précurseur de métal de transition comprend un métal de transition du groupe 8-11.

11. Procédé selon les revendications 1 ou 2, dans lequel la résine polyphénoxyde réticulée poreuse de l'étape de mise en contact a) comprend un lit fixe.

12. Procédé selon les revendications 1 ou 2, dans lequel l'étape de mise en contact et/ou l'étape d'application est conduite à une vitesse spatiale horaire en poids (WHSV) de 0,05 à 50 h⁻¹, sur la base de la quantité de la résine polyphénoxyde réticulée poreuse, et à une température de 0 °C à 250 °C.

13. Procédé selon les revendications 1 ou 2, le procédé comprenant en outre une étape d'isolement de l'acide carboxylique a,β-insaturé, ou du sel de celui-ci.

14. Procédé selon la revendication 1, comprenant en outre l'étape de régénération de la résine polyphénoxyde réticulée poreuse par mise en contact d'une résine polyphénol réticulée poreuse qui est générée à partir du procédé avec une base comprenant un cation métallique, ou par mise en contact d'une résine polyphénol réticulée poreuse qui est générée à partir du processus avec un sel contenant du métal.

15. Procédé selon la revendication 3, dans lequel le modèle particulaire basique a une solubilité dans l'eau inférieure à environ 0,25 g/l à 25 °C ou une taille moyenne de particules d'environ 2 µm (micromètres) à environ 200 pm.
